# EUROPEAN PATENT APPLICATION

(11) **EP 2 433 558 A1**
(43) Date of publication of application: **28.03.2012**
(21) Application number: 11182896.8
(22) Date of filing: 27.09.2011
(51) Int. Cl.: A61B 5/024

(54) **Biological information detector and biological information measuring device**

(30) Priority: 28.09.2010 JP 2010216726
(71) Applicant: Seiko Epson Corporation, Shinjuku-ku Tokyo 163-0811 (JP)
(72) Inventor: Yamashita, Hideto, Nagano, 392-8502 (JP)
(74) Representative: HOFFMANN EITLE

(57) **Abstract**

A biological information detector includes a light-emitting part for emitting light in an emission wavelength band that includes a wavelength of interest, a light-receiving part for receiving light R1' having biological information; a reflecting part for reflecting the light R1' having the biological information at a reflecting surface towards the light-receiving part; and a filter for transmitting the wavelength of interest, the filter being provided between a detection site and the light-receiving part. The filter inhibits light in a wavelength band from a maximum wavelength of incident light to at least a maximum wavelength in the reception wavelength band, and the reflecting surface is configured so that only light having an angle of incidence that is equal to or less than the predetermined angle reaches the light-receiving part.

## Description

### BACKGROUND

### Technological Field

The present invention relates to a biological information detector, a biological information measuring device, and the like.

### Background Technology

A biological information measuring device measures human biological information such as, for example, pulse rate, blood oxygen saturation level, body temperature, or heart rate, and an example of a biological information measuring device is a pulse rate monitor for measuring the pulse rate. Also, a biological information measuring device such as a pulse rate monitor can be installed in a clock, a mobile phone, a pager, a PC, or another electrical device, or can be combined with the electrical device. The biological information measuring device has a biological information detector for detecting biological information, and the biological information detector includes a light-emitting part for emitting light towards a detection site of a test subject (i.e., a user), and a light-receiving part for receiving light having biological information from the detection site.

A biological information detector or a biological information measuring device that can be worn on the wrist of a person can be referred to as a wrist-worn type biological information detector or biological information measuring device. A wrist-worn type biological information detector or biological information measuring device can be built into, e.g., a wristwatch (or in a broader sense, a type of electronic device worn on the wrist).

In JP 61-048338 A, there is disclosed a pulse rate monitor (or in a broader sense, a biological information measuring device). In JP 61-048338 A, a filter that only transmits wavelengths equal to or lower than 650 nm (i.e., a filter that exhibits transmission characteristics shown by a dashed line in FIG. 4 of JP 61-048338 A) is used in order to remove the effect of external light that is transmitted through the finger (e.g., external light L2 shown in FIG. 13 of JP 61-048338 A).

In JP 08-080288 A, a pulse rate monitor (or in a broader sense, a biological information measuring device) is again disclosed. In JP 08-080288 A, the wavelength of the light-emitting part of the pulse rate monitor is set to 300 nm to 700 nm (e.g., intensity characteristics of 350 nm to 600 nm shown in FIG. 4 of JP 08-080288 A), and the wavelength of the light-receiving part of the pulse rate monitor is set to 700 nm or lower (e.g., main sensitivity characteristics of 300 nm to 600 nm shown in FIG. 5 of JP 08-080288 A), in order to remove the effect of external light transmitted through the finger.

### SUMMARY

### Problems to Be Solved by the Invention

In an instance in which a pulse rate monitor such as that described in JP 61-048338 A or JP 08-080288 A is used to measure the pulse rate of a portion of the body other than a finger, such as the wrist, the effect of noise caused by external light reduces the detection accuracy of the biological information detector.

According to several aspects of the invention, it is possible to provide a biological information detector and a biological information measuring device in which the detection accuracy or the measurement accuracy can be improved.

### Means Used to Solve the Above-Mentioned Problems

A first aspect of the invention relates to a biological information detector, characterized in including
a light-emitting part for emitting light in an emission wavelength band that includes a wavelength of interest, the light being emitted towards a detection site of a test subject;
a light-receiving part for receiving light having biological information, the light being light emitted by the light-emitting part and reflected at the detection site, the light-receiving part having sensitivity in a reception wavelength band that includes the emission wavelength band;
a reflecting part having a reflecting surface, the light having the biological information being reflected at the reflecting surface towards the light-receiving part,; and
a filter for transmitting the wavelength of interest, the filter being provided between the detection site and the light-receiving part; wherein
when an angle of incidence of incident light on the filter is a predetermined angle, a maximum wavelength of the incident light passing through the filter is higher than the wavelength of interest, and the filter inhibits light that is within a wavelength band from the maximum wavelength of the incident light to at least a maximum wavelength of the reception wavelength band; and
the reflecting surface is configured so that only light having an angle of incidence that is equal to or less than the predetermined angle reaches the light-receiving part.

According to the first aspect of the invention, light within the emission wavelength band that includes the wavelength of interest (e.g., λ0), emitted from the light-emitting part (e.g., λ01 to λ02; where λ01 < λ0 < λ02), reflects at the detection site and thereby becomes the light that includes biological information. The light that includes the biological information is detected by the light-receiving part, whereby the biological information is measured. External light includes light having a variety of wavelengths including the emission wavelength band, and can represent noise when a part of the external light that has propagated through the test subject (i.e., propagated light) is received by the light-receiving part. Inhibiting the external light, which can represent noise, at the wavelength band for the filter (e.g., wavelength λ21 to wavelength λmax) makes it possible to reduce noise. Also, although the filter can transmit incident light having a wavelength that is smaller than the maximum wavelength (e.g., λ21) of incident light passing through the filter (i.e., < λ21) when the angle of incidence of incident light on the filter is at the predetermined angle, such light can be inhibited, using the reflecting surface of the reflecting part, from reaching the light-receiving part. Therefore, light that can pass through the filter and that can represent noise can be inhibited by the reflecting surface of the reflecting part. Therefore, the detection accuracy (i.e., signal-to-noise ratio) of the biological information detector improves. Inhibition of light by the filter refers to one in which a filtering effect can be obtained so that the intensity of transmitted light in relation to incident light with respect to the filter is preferably 10% or less, or further preferably, 5% or less.

According to a second aspect of the invention, the filter can be a dielectric multilayer filter.

The transmittance of the dielectric multilayer filter in relation to the wavelength of interest can be readily set to a higher level than with, e.g., transmittance of a dye absorption filter in relation to the wavelength of interest, and it becomes possible to allow light emitted by the light-emitting part to pass through more readily. Transmittance in relation to a wavelength band in the reception wavelength band (i.e., light that can represent noise) can be set to a low level, and it is possible to inhibit noise in an efficient manner.

According to a third aspect of the invention, the biological information detector further includes
a substrate formed from a material that is transparent with respect to the wavelength of interest, the light-emitting part being arranged on a first surface, and the light-receiving part being arranged on a second surface disposed opposite the first surface; and
a contact part arranged on a side towards the first surface in relation to the substrate, the contact part being formed from a material that is transparent with respect to the wavelength of interest, and the contact part having a surface that comes into contact with the test subject; wherein
the reflecting part is arranged on a side towards the second surface in relation to the substrate.

It becomes possible for the light-emitting part and the light-receiving part to overlap each other, with respect to a plan view, with the substrate interposed therebetween, and it becomes possible to reduce the size of the biological information detector.

According to a fourth aspect of the invention, the filter can be provided to at least one of the substrate, the light-receiving part, the reflecting part, and a surface of the contact part other than the contact surface.

In an instance in which the filter is provided to the contact surface, the performance of the filter can deteriorate due to an effect of oil on the test subject or other factors. Therefore, not providing the filter on the contact surface makes it possible to prevent the deterioration in the performance of the filter.

According to a fifth aspect of the invention, the maximum wavelength of the incident light can be a wavelength corresponding to a point of change at which characteristics of an increase in the intensity of external light propagating through the test subject between a peripheral side of the detection site and a center side of the detection site shift from a first incline to a second incline that is steeper than the first incline.

When some external light that has propagated through the test subject (propagated light) is received by the light-receiving part, the external light can represent noise; it has been discovered that this external light that can represent noise is light that propagates through the test subject between the peripheral side of the detection site and the center side of the detection site. Also, it has been discovered that the increase in the intensity of external light that can represent noise has a wavelength (e.g., λ1) corresponding to a point of change at which the increase shifts from a first incline (i.e., a moderate incline) to a second incline that is steeper than the first incline (i.e., a steep incline), and that light having a wavelength equal to or lower than the point of change is weak and can be disregarded in terms of noise. Setting the maximum wavelength (λ21) of incident light, when the angle of incidence of the incident light on the filter is at the predetermined angle, to the wavelength of the point of change (λ1) makes it possible to inhibit noise in an effective manner.

According to a sixth aspect of the invention, the wavelength of the point of change can be in a range of 565 nm to 595 nm.

As a result of taking activities by the test subject (e.g., a user) and other factors into consideration, the wavelength (λ1) of the point of change described above, i.e., the shortest wavelength (λ1), from among the external light that has propagated through the test subject, that can represent noise, can be set to a range of, e.g., 565 nm to 595 nm.

Another aspect of the invention relates to a biological information measuring device, including
the biological information detector described in the foregoing, and
a biological information measuring part for measuring, from a light reception signal generated in the light-receiving part, the biological information; wherein
the biological information is a pulse rate.

According to the another aspect, it is possible to increase the measurement accuracy of a biological information measuring device using a biological information detector whose detection accuracy has been improved.

### BRIEF DESCRIPTION OF THE DRAWINGS

FIG. 1 is an example of configuration of a biological information detector according to the present embodiment;

FIGS. 2A and 2B are drawings used to illustrate an entry path of external light;

FIG. 3 is an example of intensity characteristics of external light;

FIG. 4 is an example of transmission characteristics of an additional filter (i.e., comparative example);

FIGS. 5A and 5B are an example of transmission characteristics of the filter shown in FIG. 1;

FIG. 6 is an example of intensity characteristics of light emitted by the light-emitting part;

FIG. 7 is an example of intensity characteristics of light received by the light-receiving part;

FIGS. 8A and 8B are examples of reflection characteristics of the reflecting surface of the reflecting part;

FIG. 9 is an example of preferable transmission characteristics of the filter;

FIG. 10 is another example of a configuration of the biological information detector according to the present embodiment;

FIG. 11 is an example of transmission characteristics of light passing through a substrate coated with a light-transmitting film;

FIG. 12 is an example of the outer appearance of the light-transmitting film;

FIG. 13 is an example of accommodating the substrate;

FIG. 14 is another example of configuration of a biological information detector according to the present embodiment;

FIGS. 15A, 15B, and 15C are configurations of the first reflecting part;

FIGS. 16A and 16B are examples of the outer appearance of the first reflecting part and the light-emitting part;

FIG. 17 is an example of the outer appearance of the light-receiving part;

FIGS. 18A and 18B are examples of a biological information measuring device including a biological information detector;

FIG. 19 is an example of a configuration of the biological information measuring device;

FIGS. 20A and 20B are other examples of configurations of the biological information detector according to the present embodiment; and

FIG. 21 is an example of a connection between the light-receiving part and a second light-receiving part.

### DETAILED DESCRIPTION OF EXEMPLARY EMBODIMENTS

A description shall now be given for a present embodiment. The present embodiment described below is not intended to unduly limit the scope of the claims of the present embodiment. Not every configuration described in the present embodiment is necessarily an indispensible constituent feature of the invention. 1. Biological information detector

### 1.1 Example of configuration of biological information detector

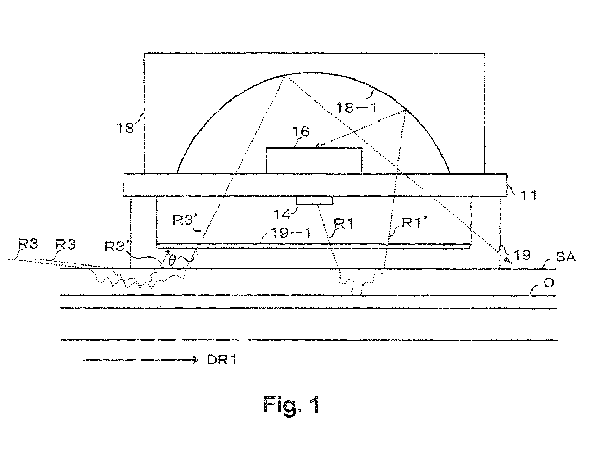

FIG. 1 is an example of configuration of a biological information detector according to the present embodiment. As shown in FIG. 1, the biological information detector includes a light-emitting part 14, a light-receiving part 16, a filter 19-1, and a reflecting part 18. The light-emitting part 14 emits light R1 towards a detection site O (e.g., a blood vessel) of a test subject (e.g., a user). The light-receiving part 16 receives light R1' (i.e., reflected light) having biological information, produced by the light R1 emitted by the light-emitting part 14 being reflected at the detection site O. The filter 19-1 is provided between the detection site O and the light-receiving part 16. The reflecting part 18 has a reflecting surface 18-1, and reflects, at the reflecting surface 18-1, the light R1' having biological information. The reflecting part 18 can have the reflecting surface 18-1 on a dome surface (i.e., a spherical surface or a paraboloid) provided on a light path between the light-emitting part 14 and the light-receiving part 16. In the example shown in FIG. 1, in cross-section view, the reflecting surface 18-1 of the reflecting part 18 describes an arc defining the spherical surface; however, the reflecting surface 18-1 can describe a parabola defining a paraboloid.

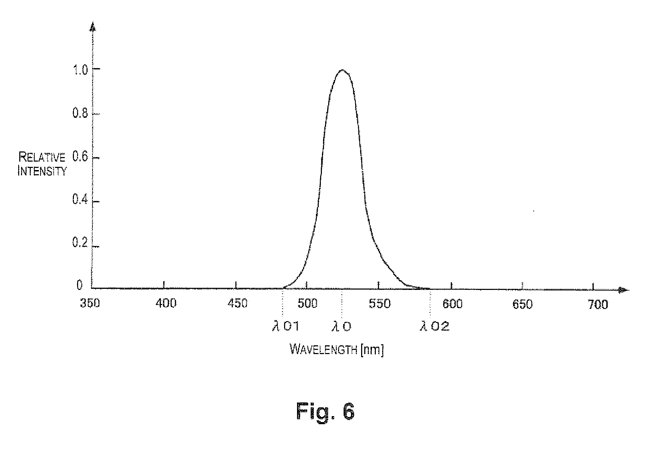

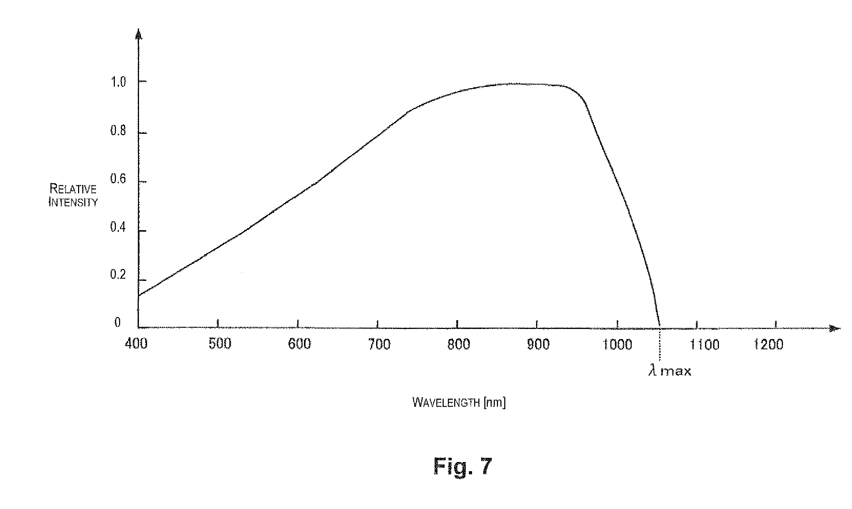

The light-emitting part 14 emits light in an emission wavelength band shown, e.g., in FIG. 6 (i.e., λ01 to λ02). Meanwhile, the light-receiving part 16 has sensitivity in a reception wavelength band (also referred to as a reception sensitivity band) shown in FIG. 6 that extends over a wide range and that includes the emission wavelength band (see FIG. 7). Thus, since the reception sensitivity band of the light-receiving part 16 (FIG. 7) is wider than the emission wavelength band of the light-emitting part 14 (FIG. 6), there is a risk of the light-receiving part 16 receiving, e.g., light outside the emission wavelength band as noise, and of the signal-to-noise ratio decreasing. The filter 19-1 is provided to inhibit light that can represent noise from being received by the light-receiving part 16. The filter 19-1 does not inhibit light at a wavelength of interest, in which biological information is reflected, from among the reflected light R1'. The wavelength of interest refers to a wavelength that is included in the emission wavelength band shown in FIG. 6 (i.e., λ01 to λ02), at which the light is absorbed by the detection site (blood vessel) O, thereby changing the intensity of the reflected light R1' (for example, λ0 : λ01 < λ0 < λ02 in FIG. 6). An example of the transmission characteristics of the filter 19-1 shall be described further below.

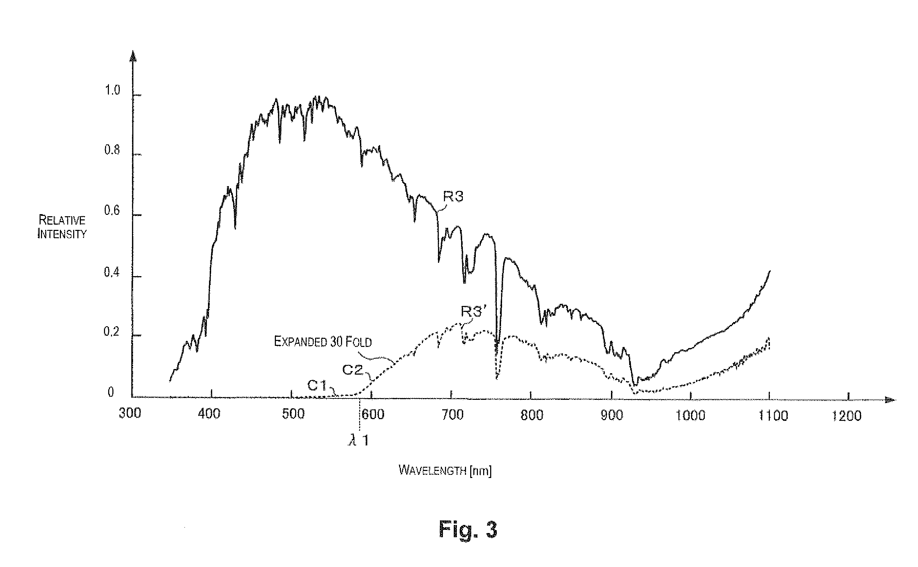

In the example shown in FIG. 1, the detection site O (e.g., a blood vessel) is in the interior of the test subject. The first light R1 proceeds into the interior of the test subject and diffuses or scatters at the epidermis, the dermis, and the subcutaneous tissue. The first light R1 subsequently reaches the detection site O and is reflected at the detection site O. The light R1' reflected at the detection site O diffuses or scatters at the epidermis, the dermis, and the subcutaneous tissue. The reflected light R1' subsequently travels towards the light-receiving part 16. The first light R1 is partially absorbed by the blood vessel. Therefore, due to an effect of the pulse, the rate of absorption at the blood vessel varies, and the amount of reflected light R1' that has been reflected at the detection site O also varies. Thus, biological information (e.g., the pulse rate) is reflected in the reflected light R1' reflected at the detection site O. More specifically, haemoglobin in the blood has an absorption coefficient in relation to light within a band of 300 nm to 700 nm that is much larger than the absorption coefficient. When the first light R1 in the emission wavelength band, whose wavelength band is within a range of, e.g., 300 nm to 700 nm in line with the light-absorbing characteristics of haemoglobin, is beamed towards the test subject, the intensity of the reflected light R1', which has been reflected at the detection site (blood vessel) O tracks the variation in blood volume and varies dramatically. Therefore, the emission wavelength band (λ01 to λ02) including the wavelength of interest (λ0) is set at least within a range of 300 nm to 700 nm. In the present embodiment, the emission wavelength band (λ01 to λ02) including the wavelength of interest (λ0) is set as shown in FIG. 6, and is set so as to not overlap with the wavelength band of external light R3', which can represent noise, shown by a dashed line in FIG. 3.

Meanwhile, the external light R3 diffuses or scatters in the interior of the test subject. The external light R3' (i.e., propagated light), which has propagated through the test subject without reaching the detection site O, travels towards the light-receiving part 16. Biological information (i.e., the pulse rate) is not reflected in the external light R3' (i.e., propagated light) which has propagated through the test subject in a first direction DR1. The external light R3 (e.g., sunlight) includes light having a variety of wavelengths. Therefore, a part of the external light R3' (i.e., propagated light) which has propagated through the test subject is inhibited by the filter 19-1 as shown in FIG. 1. However, another part of the external light R3' (i.e., propagated light) which has propagated through the test subject, e.g., R3' having an angle of incidence θ shown in FIG. 1, is transmitted through the filter 19-1. Specifically, with regards to light-obstructing characteristics of the filter 19-1, it is not possible to inhibit light of all wavelengths in the reception wavelength band from among the external light R3' which has been propagated through the test subject. Therefore, a configuration is established in which the external light R3' that could not be inhibited by the filter 19-1 is inhibited by the reflecting surface 18-1 of the reflecting part 18. An example of reflection characteristics of the reflecting surface 18-1 shall be described further below. Thus, the external light R3' (i.e., propagated light; noise), which has propagated through the test subject, is inhibited in two steps, and the detection accuracy (i.e., the signal-to-noise ratio) of the biological information detector is improved.

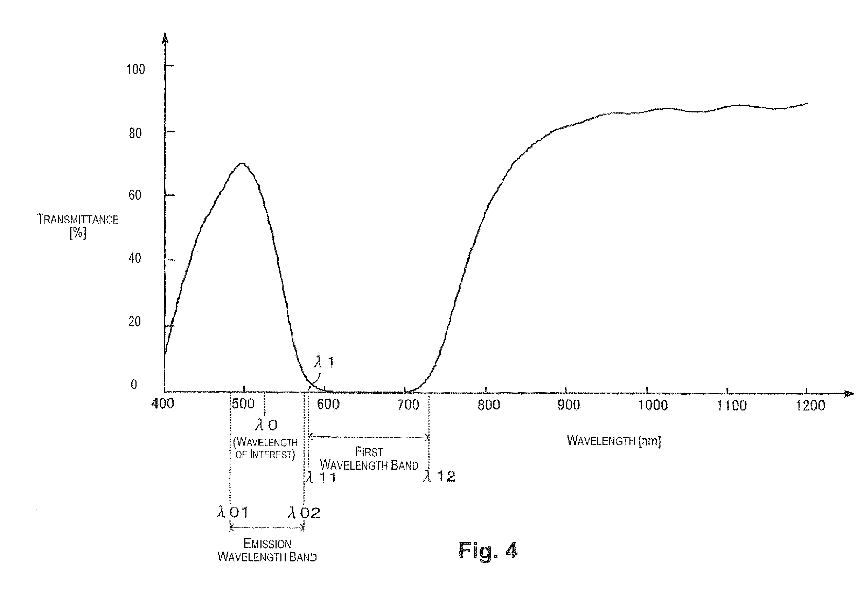

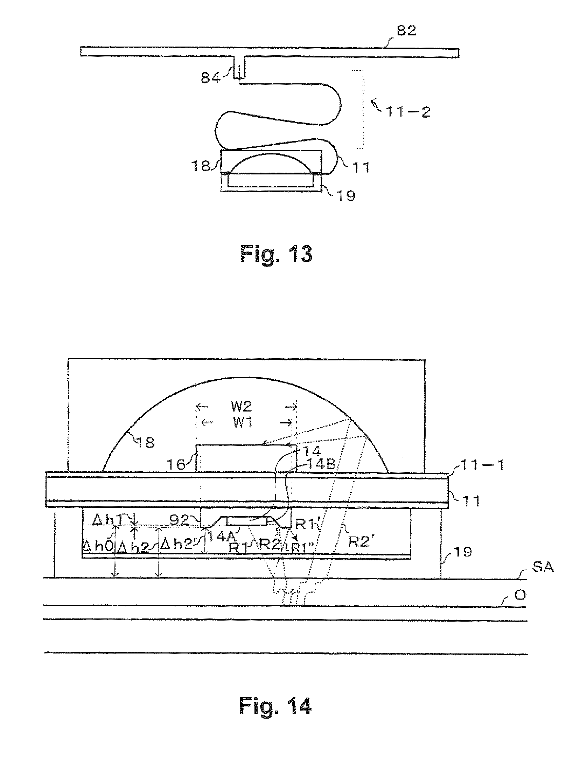

Although in Patent Citation 1, the effect of external light transmitted through a finger (e.g., external light L2 in FIG. 13 of Patent Citation 1) is considered, the effect of propagated light that has propagated through the interior of the wrist is not considered. Specifically, the present inventor(s) recognized that, in an instance in which a filter that transmits only wavelengths equal to or lower than 650 nm (i.e., a filter exhibiting transmission characteristics shown by dashed lines in FIG. 4 in Patent Citation 1) is applied, instead of the transmission characteristics of the filter 19-1 and the reflection characteristics of the reflecting surface 18-1, to a biological information detector, and in particular to a wrist-worn type biological information detector, the effect of propagated light that has propagated through the interior of the wrist cannot be disregarded.

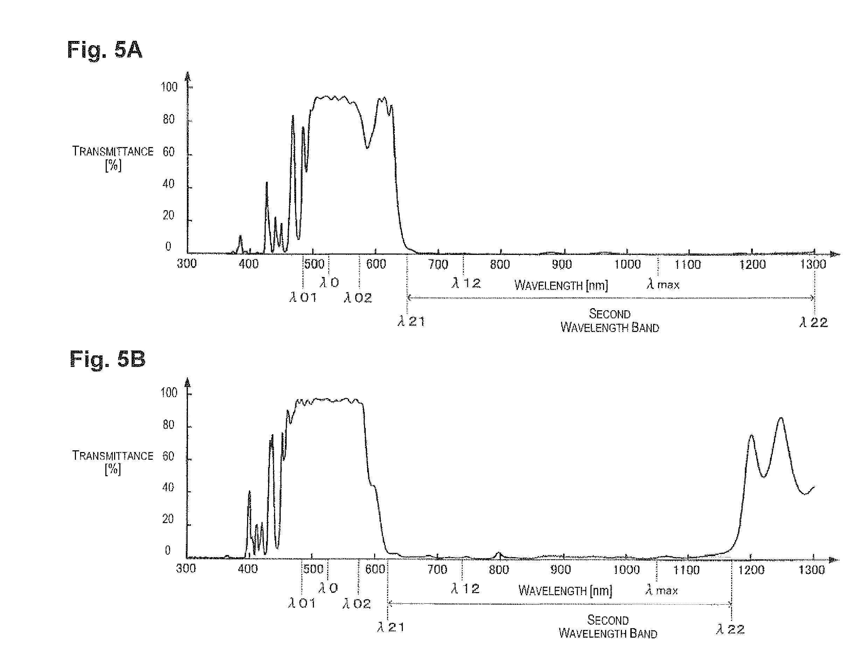

As is also the case in Patent Citation 2, the effect of external light transmitted through the finger is considered, but the effect of propagated light that has propagated through the interior of the wrist is not considered. Specifically, the present inventor(s) recognized that even if the sensitivity wavelength band of the light-receiving part 16 is set to 700 nm or lower (e.g., main sensitivity characteristics of 300 nm to 600 nm shown in FIG. 5 of Patent Citation 2), the effect of propagated light that has propagated through the interior of the wrist cannot be disregarded.

Also, as shown in FIG. 1, the biological information detector can include a contact part 19 and a substrate 11. The contact part 19 has a contact surface that comes into contact with the test subject. The light-emitting part 14 is arranged on the substrate 11 on a side towards the contact part 19, and the light-receiving part 16 is arranged on the substrate 11 on a side towards the reflecting part 18. Both of the contact part 19 and the substrate 11 are configured from a material that is transparent with respect to the wavelength of interest of the light emitted by the light-emitting part 14. The contact part 19 is formed from, e.g., glass, and the substrate 11 is formed from, e.g., polyimide.

Since the substrate 11 is sandwiched between the reflecting part 18 and the contact part 19, even if the light-emitting part 14 and the light-receiving part 16 are arranged on the substrate 11, there is no need to separately provide a mechanism for supporting the substrate 11 itself, and the number of components is smaller. Also, since the substrate 11 is formed from a material that is transparent with respect to the wavelength of interest, the substrate 11 can be arranged on the light path from the light-emitting part 14 to the light-receiving part 16, and there is no need to accommodate the substrate 11 at a position away from the light path, such as within the reflecting part 18. Thus, it is possible to provide a biological information detector that can be readily assembled. Also, the reflecting part 18 is capable of increasing the amount of light reaching the light-receiving part 16, and the detection accuracy (i.e., signal-to-noise ratio) of the biological information detector is improved.

The thickness of the substrate 11 is e.g., 10 µm to 1000 µm. Wiring for the light-emitting part 14 and wiring for the light-receiving part 16 can be formed on the substrate 11. The substrate 11 is, e.g., a printed circuit board; however, a printed circuit board is not generally formed from a transparent material. Specifically, the inventors purposefully used a configuration in which the printed circuit board is formed from a material that is transparent at least with respect to the wavelength of interest from the light-emitting part 14. The thickness of the protecting part 19 is, e.g., 1 µm to 3000 µm.

Examples of configurations of the biological information detector are not limited by that shown in FIG. 1, and the shape, or a similar attribute, of a part of the example of configuration (e.g., the reflecting part 18) can be modified. The biological information can also be blood oxygen saturation level, body temperature, heart rate, or a similar variable; and the detection site O can be positioned at a surface SA of the test subject. In the example shown in FIG. 1, the first light R1 is shown by a single line; however, in reality, the light-emitting part 14 emits many rays of light in a variety of directions. Also, the external light R3 is shown by two lines; however, in reality, many external rays of light enter the interior of the test subject from a variety of directions, and as a result, in reality, many external rays of light R3' that have propagated through the test subject travel towards the biological information detector.

### 1.2 Effect of external light

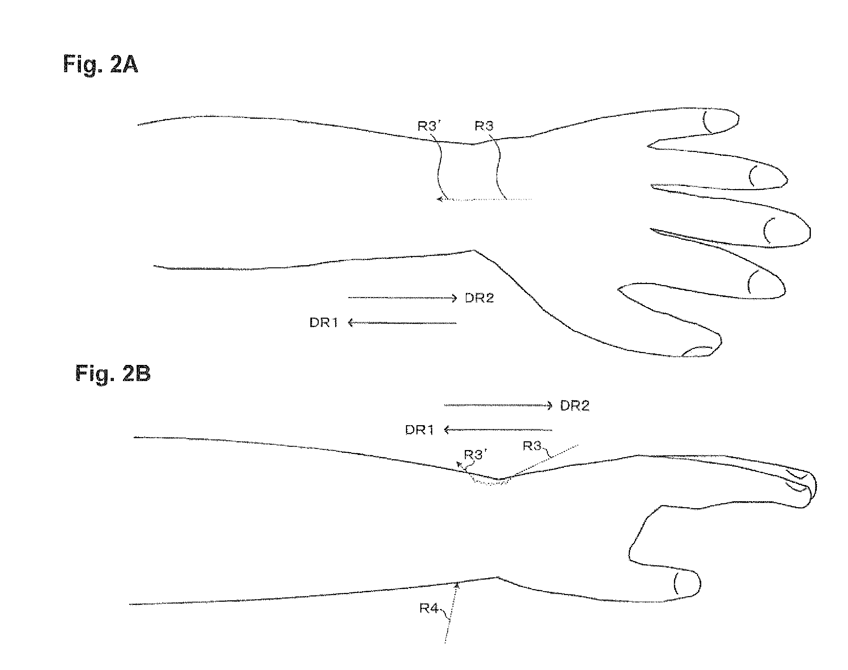

FIGS. 2A and 2B are drawings used to illustrate an entry path of external light. While many external rays of light enter the interior of the test subject from a variety of directions, external light R3 enters the interior of the test subject from, e.g., a first direction DR1 extending from a peripheral side (e.g., the side towards the back of the hand) of the detection site O towards a center side (top of arm) with respect to a plan view as shown in FIG. 2A. The external light R3 diffuses or scatters in the interior of the test subject, at, e.g., a location at which a tendon, a bone, or a similar obstacle is not present. External light can also enter the interior of the test subject from, e.g., a second direction extending from the center side to the peripheral side of the detection site O. As shown in FIG. 2B, external light R3' (propagated light) which has propagated through the test subject in the first direction DR1 returns to the exterior of the test subject. The present inventor(s) recognized that propagated light of such description causes noise in a biological information detector, and in particular to a wrist-worn type biological information detector. Meanwhile, external light R4 travelling from the palmar side to the dorsal side of the wrist is barely transmitted through the interior of the test subject. The present inventor(s) recognized that there is no need to consider the effect of the external light R4 travelling from the palmar side to the dorsal side of the wrist.

The solid line in FIG. 3 shows an example of intensity characteristics of sunlight (or in a broader sense, external light R3). In the example shown in FIG. 3, the intensity of external light R3 is at a maximum at a wavelength of 530 nm. The dotted line in FIG. 3 shows sunlight produced by the sunlight R3 shown by the solid line propagating through the interior at the surface of the wrist (or in a broader sense, external light R3' which has propagated through the test subject), and this sunlight R3 can represent noise in relation to the light-receiving part 16. The intensity of the sunlight R3' which has propagated through the interior of the surface of the wrist is much smaller than that of the sunlight R3 itself, and has therefore been expanded 30-fold in the example in FIG. 3.

As shown in FIG. 3, the sunlight R3' which has propagated through the interior of the surface of the wrist is at a maximum at a wavelength of 710 nm. Also, sunlight R3' which has propagated through the interior of the surface of the wrist has a wavelength that is less than 700nm, which is the lower limit of the biological window (i.e., from 700 nm to 110 nm). Also, the intensity of sunlight (propagated light) R3' having a wavelength of 690 nm is 90% of the maximum (i.e., for 710 mn); the intensity of sunlight (propagated light) R3' having a wavelength of 650 nm is 50% of the maximum; and the intensity of sunlight (propagated light) R3' having a wavelength of 600 nm is 20% of the maximum. This means that, with a filter taught in Patent Citation 1 that transmits only wavelengths equal to or lower than 650 nm, the effect of sunlight (propagated light) R3' cannot be disregarded, and with a light-receiving part taught in Patent Citation 2 having a sensitivity wavelength band of 700 nm or lower, the effect of sunlight (propagated light) again cannot be disregarded.

Also, the intensity of sunlight (propagated light) R3' having a wavelength of 590 nm is 10% of the maximum (i.e., at 710 nm), and the intensity of sunlight (propagated light) R3' having a wavelength of 580 nm is 5% of the maximum. In particular, a wavelength of 580 nm for the sunlight R3' (i.e., propagated light) shown by a dotted line in the example shown in FIG. 3 is the wavelength of the point of change (λ1) at which the characteristics of an increase in the intensity of external light propagating through the interior of the surface of the wrist shifts from a moderate incline (i.e., first incline) C1 to a steep incline (i.e., second incline) C2. The amount of sunlight (propagated light) R3' having a wavelength of 580 nm or lower is less than 5%; i.e., negligible, compared to the intensity of the sunlight (propagated light) R3' as a whole. Therefore, external light R3' having a wavelength of 580 nm, which is the wavelength of the point of change (λ1), or higher is one that can represent noise in relation to the light-receiving part 16. Light having a wavelength of 580 nm, which is the wavelength of the point of change (λ1), can be referred to as the shortest wavelength, from among the external light R3' which has propagated through the test subject, that can represent noise. As a result of the present inventor(s) considering the intensity characteristics of sunlight (propagated light) shown in FIG. 3, it was found that it is important to effectively inhibit the sunlight (propagated light) R3' having a wavelength of 580 nm or higher, which is much higher than the emission wavelength band (i.e., maximum range in FIG. 6 is 485 nm to 575 nm). The wavelength of the point of change (λ1) need only be higher than the wavelength of interest (λ0), and can be included in the emission wavelength band.

Activities by the test subject (i.e., the user) and other factors can cause a variation in the ease of bonding between oxygen and haemoglobin in the blood, and the shortest wavelength, from among the external light R3' which has propagated through the test subject, that can represent noise (i.e., 580 nm) can vary from 565 nm to 595 nm. Therefore, it is possible to set, as appropriate, the wavelength of interest, the emission wavelength band, or the light-inhibiting characteristics of the filter 19-1, in accordance with the wavelength band of light to be inhibited or removed.

### 1.3 Comparative example and transmission characteristics of filter (light-inhibiting characteristics)

Although FIG. 1 shows the filter 19-1 as the filter, an additional filter (not shown) can be provided between the detection site O and the light-receiving part 16 and a comparative example can be configured. External light R3' that could not be inhibited using the filter 19-1 can be inhibited using an additional filter 15. A comparative example of such description is not one that is publicly known, at the time of filing, among those skilled in the art; and is a novel aspect at the time of filing of this application. The additional filter (not shown) is provided between the detection site O and the light-receiving part 16, and can, e.g., be arranged on the substrate 11. The additional filter transmits light at the wavelength of interest (λ0 : λ01 < λ0 < λ02) within the emission wavelength band (λ01 to λ02) shown in FIG. 6, and inhibits light in a first wavelength band (λ11 to λ12 : λ0 < λ11 < λ12, shown in FIG. 4), within the reception wavelength band, which is higher than the wavelength of interest (λ0). The first wavelength band is a band from a wavelength λ11 to a wavelength λ12. The wavelength λ11 is equal to or higher than the wavelength of the point of change (λ1) at which the characteristics of an increase in the intensity of the external light R3' propagating through the test subject between the peripheral side of the detection site O and the center side of the detection site O shifts from the moderate incline C1 to the steep incline C2 shown in FIG. 3 (i.e., λ0 < λ11 ≤λ1).

The filter 19-1 transmits light at the wavelength of interest (λ0) and inhibits light in a second wavelength band (λ21 to λ22 in FIGS. 5A and 5B). The lower limit of the second wavelength band (λ21) is also a maximum wavelength of light passing through the filter 19-1. The lower limit of the second wavelength band (λ21) is equal to or higher than the first wavelength (λ21), and the upper limit (λ22) is a second wavelength (λ22) that is higher than the first wavelength (λ21) and equal to or higher than the emission wavelength band (maximum wavelength λmax in FIG. 7).

Thus, in the comparative example, the filter 19-1 and the additional filter is able to transmit the wavelength of interest λ0 within the emission wavelength band, and to inhibit all light within the reception sensitivity band having a wavelength equal to or higher than the shortest wavelength (λ1) of the propagated light R3' which can represent noise.

### 1.3.1 Specific example of characteristics of the additional filter

FIG. 4 shows an example of transmission characteristics of the additional filter. In the example shown in FIG. 4, the additional filter is a dye absorption filter. In the example shown in FIG. 4, the transmittance of light having a wavelength within a range of 600 nm to 700 nm is 0%. The transmittance of light having a wavelength within a range of 580 nm to 730 nm is 50% or less. The transmittance of light having a wavelength within a range of 565 nm to 740 nm is 10% or less. The first wavelength band, in which the additional filter exhibits light-inhibiting characteristics, preferably corresponds to a transmittance of 10% or less. However, in the comparative example, a region from, e.g., 580 nm to 730 nm, which is a wavelength band in which the transmittance is 5% or less, is referred to as the first wavelength band. This is because if the transmittance is preferably 10% or less, and further preferably 5% or less, the fact that the intensity of the external light R3 propagating through the interior of the body is already weak means that even if the light is received by the light-receiving part 16 after being filtered by transmittance of 10% or less, the signal-to-noise ratio can be maintained to a relatively high level. Thus, the additional filter is capable of inhibiting light in the first wavelength band (e.g., wavelength λ11 = 580 nm to wavelength λ12 = 730 nm). Setting the first wavelength band so as to start from at least the shortest wavelength that can represent noise (i.e., 580 nm) from among the external light R3' (propagated light) makes it possible to inhibit the effect of sunlight (propagated light), which has not been considered in Patent Citations 1 and 2.

In relation to the sunlight (propagated light) R3' shown by a dotted line in the example of FIG. 3, intensity of light having a wavelength of 700 nm or higher is greater than the intensity of light having a wavelength in the vicinity of 600 nm. Therefore, in the example shown in FIG. 4, the effect of transmittance for the light R3' having a wavelength of 700 nm or higher is greater than the effect of transmittance for light having a wavelength in the vicinity of 600 nm. In relation to transmission characteristics of the additional filter (i.e., dye absorption filter) in FIG. 4, the transmittance of light R3' having a wavelength of 700 nm or higher increases. Therefore, the filter 19-1 can be provided in order to inhibit the light R3' having a wavelength of 700 nm or higher in particular.

The dye absorption filter can be formed from, e.g., a gelatine resin, a polyester resin, or another resin into which an absorption-type dye has been mixed; and can be a resin sheet. A dye absorption filter formed from a resin sheet can be used, e.g., for a stage illumination device for emitting a specific color. The dye absorption filter can also be formed from a plastic (i.e., synthetic resin) into which an absorption-type resin has been mixed, and can be used, e.g., for sunglasses lens.

In the comparative example, the additional filter (dye absorption filter) can be used alongside the filter 19-1 to inhibit the effect of sunlight (propagated light). However, as shown in FIG. 4, the transmittance at the wavelength of interest λ0 is about 60%, and the present inventor(s) recognized the need to increase the transmittance at the wavelength of interest λ0. In the present embodiment, an improvement is made to the reflection characteristics (i.e., structure) of the reflecting surface 18-1 of the reflecting part 18, instead of the additional filter. An example of the reflection characteristics of the reflecting surface 18-1 shall be described further below.

### 1.3.2 Specific example of characteristics of filter in FIG. 1

FIGS. 5A and 5B show an example of transmission characteristics of the filter 19-1 in FIG. 1. The filter 19-1 can be used on its own in the present embodiment, and can be used alongside the additional filter in the comparative example. In the example shown in FIGS. 5A and 5B, the filter 19-1 is a dielectric multilayer filter. In FIG. 5A, light (i.e., incident light) is caused to be transmitted orthogonally with respect to the filter 19-1, and the angle of incidence θ is 0°. In FIG. 5B, light is caused to be transmitted diagonally with respect to the filter 19-1, and the angle of incidence θ of the incident light is 45°. As shown in FIGS. 5A and 5B, the transmittance of the dielectric multilayer filter is dependent on the angle of incidence θ of the light.

In the example shown in FIG. 5A, transmittance of light having a wavelength in the range of 670 nm to 1300 nm is 1% or less. Transmittance of light having a wavelength in the range of 650 nm to 1300 nm is 5% or less. Transmittance of light having a wavelength in the range of 645 nm to 1300 nm is 10% or less. As with the additional filter, a range of 650 m to 1300 mn, which is a wavelength band at which the transmittance is equal to or less than 5% in an instance in which the angle of incidence θ is 0°, is referred to as a second wavelength band. Again, this is because if the transmittance is, e.g., 5% or less, since the intensity of the external light R3 propagating through the interior of the body is already weak, even if the light is received by the light-receiving part 16, the signal-to-noise ratio can be maintained to a relatively high level. Thus, the filter 19-1 is capable of inhibiting light in the second wavelength band (e.g., from the first wavelength λ21 = 650 nm to the second wavelength λ22 = 1300 nm). In the comparative example, inhibiting light in the second wavelength band extending from at least the upper limit of the first wavelength band (e.g., wavelength λ12 = 740 nm) to the second wavelength λ22 which is equal to or higher than the maximum wavelength λmax of the reception wavelength band shown in FIG. 7 makes it possible to inhibit the effect of sunlight (propagated light) R3', which cannot be inhibited using the additional filter. Furthermore, causing a part of the first and second wavelength bands, in which the filter 19-1 and the additional filter inhibit light, to overlap makes it possible, even in an instance in which the characteristics of one of the filters exhibits an attenuation slope (in particular, an attenuation slope in a band of 700 nm to 800 nm in the additional filter), for the other filter having overlapping light-inhibiting characteristics to inhibit light in the region corresponding to the attenuation slope.

In the example of FIG. 4 showing the characteristics of the alternative filter, the transmittance for wavelengths in the vicinity of 700 nm exceeds 1%. Therefore, in the comparative example, in relation to the transmission characteristics of the filter 19-1 (i.e., dielectric multilayer filter), the transmittance for wavelengths in the vicinity of 700 nm is preferably set to, e.g., 1% or less, as shown in FIG. 5A.

In the example shown in FIG. 5B, which shows characteristics for an angle of incidence θ of 45°, transmittance of light having a wavelength in a range of 620 nm to 1170 nm is 5% or less. Transmittance of light having a wavelength in a range of 615 nm to 1175 nm is 10% or less. A range of 620 nm to 1170 nm, which is a wavelength band in which the transmittance is 5% or less in an instance in which the angle of incidence θ is 45°, is referred to as a second wavelength band. Again, this is because if the transmittance is, e.g., 5% or less, the fact that the intensity of the external light R3 propagating through the interior of the body is already weak means that even if the light is received by the light-receiving part 16, the signal-to-noise ratio can be maintained to a relatively high level. Thus, even in an instance in which the angle of incidence θ is 45°, the filter 19-1 is capable of inhibiting light in the second wavelength band (e.g., from λ21 = 620 nm to the third wavelength λ22 = 1170 nm). In the comparative example, even if the angle of incidence θ is 45 °, inhibiting light in the second wavelength band extending from at least the upper limit of the first wavelength band (e.g., wavelength λ12 = 740 nm) to the third wavelength λ22 which is equal to or higher than the maximum wavelength λmax of the reception wavelength band shown in FIG. 7 makes it possible to inhibit the effect of sunlight (propagated light) R3', which cannot be inhibited using the additional filter.

The dielectric multilayer filter can be formed by layering a plurality of dielectric layers. The dielectric layers are, e.g., formed by alternately accumulating a first dielectric layer and a second dielectric layer several times, using, e.g., a sputtering device. The refractive index of the first dielectric layer (e.g., a TiO₂ layer) is higher than the refractive index of the second dielectric layer (e.g., an SiO₂ layer). Interference of the reflected light, which occurs at the interface between the first dielectric layer and the second dielectric layer makes it possible for the dielectric multilayer filter to inhibit light in the second wavelength band.

In the present embodiment, an improvement is made to the reflection characteristics of the reflecting surface 18-1 of the reflecting part 18, instead of the alternative filter. In an instance in which the alternative filter (with transmittance at wavelength of interest λ0 of about 60%) is not used, in the example shown in FIG. 5A and 5B, the transmittance of the filter 19-1 at the wavelength of interest λ0 exceeds 90%, and it is possible to maintain a high signal-to-noise ratio.

As a result of consideration by the inventors with regards to the transmission characteristics in FIG. 5B, it was found that configuring the reflecting surface 18-1 so that only light having an angle of incidence θ equal to or less than 45° reaches the light-receiving part 16 makes it possible to avoid the attenuation in the comparative example in the emission wavelength band (i.e., λ01 to λ02) including the wavelength of interest λ0. However, in the example shown in FIG. 5B, the effect of external light (propagated light) which has propagated through the test subject is present, and it is preferable in the present embodiment to set the first wavelength λ21 when the angle of incidence θ of incident light on the filter 19-1 is at the predetermined angle to the shortest wavelength (i.e., 580 nm) that can represent noise from among the external light R3' which has propagated through the test subject. An example of preferable transmission characteristics of the filter 19-1 will be described further below. The first wavelength λ21 when the angle of incidence θ of incident light on the filter 19-1 is at the predetermined angle is the maximum wavelength that is transmitted through the filter when the angle of incidence θ of the incident light on the filter is at the predetermined angle, and can be referred to as the cut-off wavelength.

### 1.4. Emission wavelength band and wavelength of interest

FIG. 6 shows an example of intensity characteristics of the light emitted by the light-emitting part 14. In the example shown in FIG. 6, the intensity is at a maximum for light having a wavelength of 525 nm, and the intensity of light having other wavelengths is normalized with respect thereto. Also, in the example shown in FIG. 6, the wavelengths of light emitted by the light-emitting part 14 are within a range of 485 nm to 575 nm. In the example shown in FIG. 6, the relative intensity of light having wavelengths in a range of 505 nm to 540 nm is 0.5 (= 50%) or above. Specifically, the half-width of light emitted by the light-emitting part 14 is 30 nm. Also, the relative intensity of light having wavelengths in a range of 500 nm to 555 nm is 0.1 (= 10%) or above. The wavelength of interest of light emitted by the light-emitting part 14 can be 525 nm corresponding to the maximum value, and a range of, e.g., 500 nm to 555 nm can be set to the wavelength of interest of light emitted by the light-emitting part 14. In essence, the wavelength of interest λ0 is included in the emission wavelength band (i.e., λ01 to λ02), is a wavelength at which absorption takes place at the detection site O and the reflection intensity varies (or in a broader sense, a wavelength at which biological information is included), and is a wavelength of light that is not inhibited at the filter 19-1.

In relation to transmission characteristics of the alternative filter shown in FIG. 4, the transmittance of light in a range of 500 nm to 555 nm, which is a wavelength band centered around the wavelength of interest λ0 in the emission wavelength band, is 25%, which is higher than the transmittance of the first wavelength band (e.g., 5% or less). The transmittance of light at the wavelength of interest λ0 is 40% or above. Also, in relation to the transmission characteristics of the filter 19-1 shown in FIG. 5A, transmittance of light in a range of 500 nm to 555 nm, which is a wavelength band centered around the wavelength of interest λ0 in the emission wavelength band, is 85% or above, which is higher than the transmittance of the second wavelength band (e.g., 5% or less). Thus, both of the alternative filter and the filter 19-1 is capable of transmitting a part or all of the light in the emission wavelength band, including at least the wavelength of interest λ0, emitted by the light-emitting part 14. In the comparative example, light at the wavelength of interest λ0 propagated through the alternative filter attenuates, and it is therefore preferable that the filter 19-1 exhibits a high transmittance with respect to the wavelength of interest λ0 as shown in FIG. 5A. In the present embodiment, it is generally also preferable that the filter 19-1 exhibits a high transmittance with respect to the wavelength of interest λ0.

The light-emitting part 14 is, e.g., an LED. The light emitted by the LED can have a maximum intensity (or in a broader sense, a peak value) in a wavelength band at which the detection site O exhibits light-absorbing characteristics, e.g., in a range of 425 nm to 625 nm, and can be, e.g., green. The thickness of the light-emitting part 14 is, e.g., 20 µm to 1000 µm.

### 1.5 Reception wavelength band

FIG. 7 shows an example of sensitivity characteristics of light received by the light-receiving part 16. In the example shown in FIG. 7, the sensitivity is at a maximum for light having a wavelength of 875 nm, and the sensitivity with respect to light having other wavelengths is normalized thereto. In the example shown in FIG. 7, the light-receiving part 16 is a Si photodiode. The light-receiving part 16 is not limited to the example shown in FIG. 7, and can be another Si photodiode having a maximum (or in a broader sense, a peak value) sensitivity in a range of, e.g., 800 nm to 1000 nm. The light-receiving part 16 can also be a GaAsP photodiode or another photodiode having a maximum (or in a broader sense, a peak value) sensitivity in a range of, e.g., 550 nm to 650 nm. The thickness of the light-receiving part 16 is, e.g., 20 µm to 1000 µm. A Si photodiode is less costly than a GaAsP photodiode, but perceives infrared light; therefore, in the comparative example, the filter 19-1 is necessary in addition to the alternative filter.

In the example shown in FIG. 7, the relative sensitivity for light having a wavelength of 1050 nm is 0.1 (= 10%). The sensitivity wavelength band of the light-receiving part 16 can be set so that the relative sensitivity is 0.1 or above, and can be in a range of 380 nm to 1050 nm. The second wavelength band (or in a broader sense, a filter band) of the filter 19-1 can end at the upper limit of the sensitivity wavelength band of the light-receiving part 16 (i.e., λmax = 1050 nm).

### 1.6 Arrangement of filter

In the example shown in FIG. 1, the filter 19-1 is provided on a light path between the detection site O and the light-receiving part 16. In the example shown in FIG. 1, the external light R3' (propagated light) and the received light R1' originate in different places, but the external light R3 propagates to the test subject in the vicinity of the detection site O. Specifically, providing the filter 19-1 on the light path between the detection site O and the light-receiving part 16 makes it possible to inhibit the external light R3' (propagated light). In a wrist-worn type biological information detector, external sunlight or other external light R3 readily enters the biological information detector due to the movement of the wrist when the user is walking or otherwise moving, and the presence of the filter 19-1 is therefore important.

In the example shown in FIG. 1, the filter 19-1 is arranged on the contact part 19. In an instance in which the filter 19-1 is a dielectric multilayer filter exhibiting transmission characteristics shown in FIG. 5A, the wavelength of interest of light emitted by the light-emitting part 14 is transmitted through the filter 19-1, and is not attenuated by any significant degree. Therefore, the second filter 19-1 can be provided on the light path between the light-emitting part 14 and the detection site O. The contact part 19 has a contact surface in contact with the test subject, and the contact surface is smooth. In an instance in which the filter 19-1 is a dielectric multilayer filter, multiple dielectric layers can be readily layered on the contact part 19 whose smoothness is high. Specifically, the dielectric multilayer filter 19-1, which is dependent on the angle of incidence θ, is capable of inhibiting infrared light in a more effective manner with increasing smoothness. However, in an instance in which the dielectric multilayer filter is arranged on the contact surface of the contact part 19, the effect of oil on the test subject or other factors cause the performance of the dielectric multilayer filter to deteriorate. Therefore, the filter 19-1 is preferably provided on the light path between the detection site O and the light-receiving part 16 other than the contact surface of the contact part 19. For example, the filter 19-1 is preferably arranged on a surface disposed opposite the contact surface (i.e., an opposing surface), as shown in FIG. 1. The filter 19-1 can also be arranged on the entirety of an inner wall of the contact part 19.

### 1.7 Reflection characteristics of reflecting surface

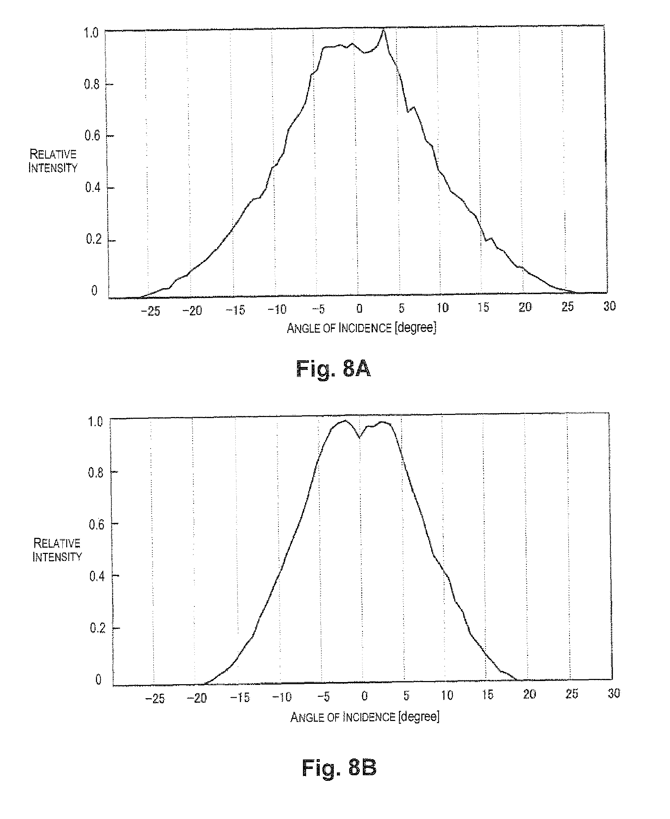

FIGS. 8A and 8B show examples of reflection characteristics of the reflecting surface 18-1 of the reflecting part 18. In the example shown in FIG. 1, in cross-section view, the reflecting surface 18-1 of the reflecting part 18 describes an arc defining a spherical surface. In the example shown in FIG. 8A, the curvature radius of the arc is 2.55 mm, and the height of the arc is 1.25 mm. In the example shown in FIG. 8B, the curvature radius of the arc is 3.5 mm, and the height of the arc is 1.7 mm. In FIGS. 8A and 8B, in an instance in which the intensity of the incident light on the reflecting part 18 is set to the maximum value, the intensity of the light reflected at the reflecting surface 18-1 is normalized with respect thereto. Angles of incidence in the first direction DR1 shown in FIG. 1 can be referred to as, e.g., positive angles of incidence, and angles of incidence in the direction opposite to the first direction DR1 can be referred to as, e.g., negative angles of incidence.

In the example shown in FIG. 8A, it can be seen that only light having an angle of incidence θ (absolute value) equal to or less than 26° can reach the light-receiving part 16. In the example shown in FIG. 8B, it can be seen that only light having a angle of incidence θ (absolute value) equal to or less than 19° can reach the light-receiving part 16. Thus, the structure (i.e., shape) of the reflecting surface of the reflecting part 18 makes it possible to inhibit light having an angle of incidence greater than a predetermined angle from reaching the light-receiving part 16. Reflection characteristics of the reflecting surface 18-1 are not limited to the examples shown in FIGS. 8A and 8B. For example, the curvature radius or the height of the arc can be adjusted, thereby adjusting the predetermined angle. The reflecting surface 18-1 of the reflecting part 18 is not limited to an arc defining a spherical surface in cross-section view; the predetermined angle can be adjusted when another shape is used.

### 1.8 Preferable transmission characteristics of filter (light-inhibiting characteristics)

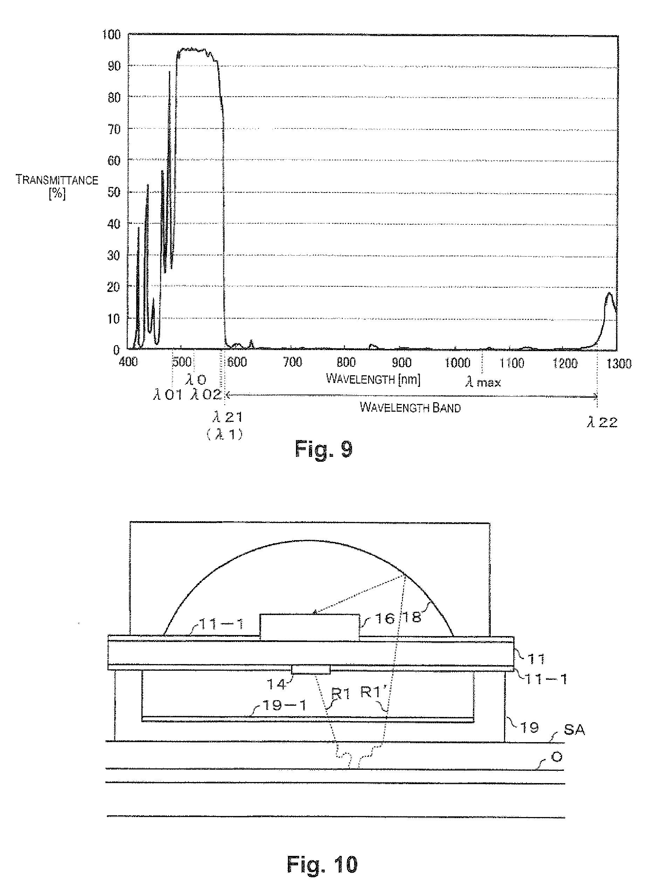

FIG. 9 shows an example of preferable transmission characteristics of the filter 19-1. In the example shown in FIG. 9, the filter 19-1 is a dielectric multilayer filter. Light is transmitted through the filter 19-1 diagonally, and the angle of incidence θ is 30°. The first wavelength λ21 (i.e., the cut-off wavelength) when the angle of incidence θ of incident light on the filter 19-1 is 30° can be set to the shortest wavelength (i.e., 580 nm) that can represent noise from among the external light R3' propagated through the test subject. It is thereby possible to inhibit noise in an effective manner. Also, in an instance in which a filter 19-1 having transmission characteristics as shown in FIG. 9 is used, the reflecting surface 18-1 is preferably designed so that only incident light having an angle of incidence θ equal to or less than 30° reaches the light-receiving part 16.

### 1.9 Another example of configuration of biological information detector

FIG. 10 shows another example of a configuration of the biological information detector according to the present embodiment. As shown in FIG. 10, a light-transmitting film 11-1 can be formed on a first surface (e.g., a front surface), and a second surface disposed opposite the first surface (e.g., a reverse surface) of the substrate 11. Structures that are identical to the example described further above are affixed with the same numerals, and a description of the structures shall not be provided. The light-transmitting film 11-1 can also be formed on the first surface only, or formed on the second surface only. In the example shown in FIG. 10, the light-transmitting film 11-1 is formed on a light-transmitting region of the substrate 11 where the light-emitting part 14 and the light-receiving part 16 are not arranged. The light-transmitting film 11-1 can be formed from, e.g., a solder resist (or in a broader sense, a resist).

In the example shown in FIG. 10, wiring for the light-emitting part 14 and wiring for the light-receiving part 16 are not shown. However, the first surface and the second surface of the substrate 11 can be processed so as to have a rough surface so that wirings on the substrate 11 do not peel off. Therefore, the light-transmitting film 11-1 is formed on the first surface and the second surface, whereby the roughness on the surface of the substrate 11 is filled with the light-transmitting film and the smoothness of the entire substrate 11 is increased. Specifically, the light-transmitting film 11-1 on the substrate 11 is smooth, and can therefore reduce dispersion of light on the roughness on the surface of the substrate 11 during transmission of the light through the substrate 11. Specifically, the presence of the light-transmitting film 11-1 increases the transmittance of the substrate 11. Therefore, the amount of light reaching the light-receiving part 16 increases and the detection accuracy of the biological information detector increases further.

The refractive index of the light-transmitting film 11-1 is preferably between the refractive index of air and the refractive index of the substrate 11. Further preferably, the refractive index of the light-transmitting film 11-1 is preferably closer to the refractive index of the substrate 11 than the refractive index of air. In such an instance, it is possible to reduce reflection of light on the interface.

### 1.10 Light-transmitting substrate

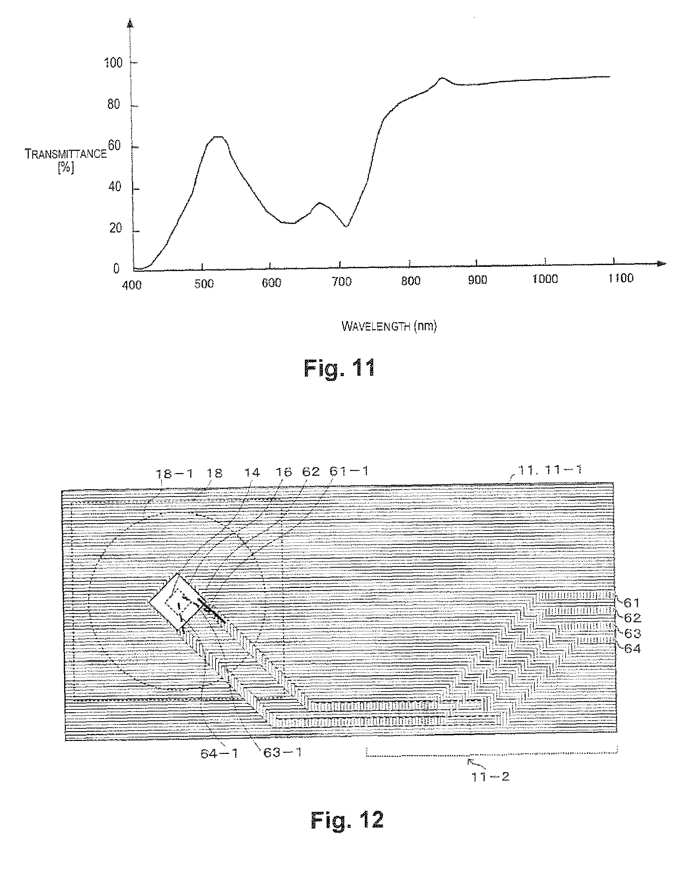

FIG. 11 shows an example of transmission characteristics of light passing through the substrate 11 coated with the light-transmitting film 11-1. In the example shown in FIG. 11, transmittance is calculated using the intensity of light before being transmitted through the substrate 11 and the intensity of light after being transmitted through the substrate 11. In the example shown in FIG. 11, in a region of wavelengths equal to or less than 700 nm, which is the lower limit of the biological window, the transmittance is at a maximum for light having a wavelength of 525 nm. Or, in the example shown in FIG. 11, in the region of wavelengths equal to or less than 700 nm, which is the lower limit of the biological window, the maximum transmittance of light passing through the light-transmitting film 11-1 falls within a range of ±10% of the maximum intensity of light having certain wavelength generated by the light-emitting part 14. It is preferable that the light-transmitting film 11-1 thus selectively transmits light generated by the light-emitting part 14 (e.g., the first light R1 in FIG. 10 (or in a narrower sense, the reflected light R1' produced by the first light R1 being reflected)) The presence of the light-transmitting film 11-1 makes it possible to enhance the smoothness of the substrate 11 and prevent, to a certain extent, a decrease in efficiency of the light-receiving part 16. In an instance in which transmittance has a maximum value (or in a broader sense, a peak value) within, e.g., a visible light region for light having a wavelength of 525 nm, as shown in the example in FIG. 11, the light-transmitting film 11-1 is, e.g., green.

FIG. 12 shows an example of an outer appearance, with respect to a plan view, of the light-transmitting film 11-1 shown in FIG. 10. As shown in FIG. 12, with respect to the plan view (when viewed from, e.g., towards the light-receiving part 16 in FIG. 10), the substrate 11 on which the light-transmitting film 11-1 is formed is rectangular. In the example shown in FIG. 12, the light-receiving part 16 is placed on the first surface (e.g., the front surface) of the substrate 11. The light-transmitting film 11-1 can be formed on a region of the first surface of the substrate 11 on which the light-receiving part 16 is not placed.

Specifically, a wiring 61 that connects to, e.g., an anode of the light-receiving part 16 is also formed, and a wiring 62 that connects to, e.g., a cathode of the light-receiving part 16 is also formed, on the first surface of the substrate 11. In the example shown in FIG. 12, the wiring 61 is connected to the anode of the light-receiving part 16 via, e.g., a bonding wire 61-1, and the wiring 62 is directly connected to the cathode of the light-receiving part 16. The light-transmitting film 11-1 can be applied on the first surface of the substrate 11 after the wiring 61 and the wiring 62 are formed on the substrate 11. Specifically, the light-transmitting film 11-1 can be formed on the wiring 61 and the wiring 62. However, the substrate 11 can also be selectively applied only on a region of the substrate 11 on which the light-receiving part 16, the wiring 61, and the wiring 62 are not placed (i.e., on the light-transmitting region).

Then, the reflecting part 18 can be formed or secured in place on the substrate 11 (and the light-transmitting film 11-1). As shown in FIG. 6, with respect to the plan view, the reflecting part 18 has a square profile, and the boundary of the reflecting surface 18-1 (dome surface) of the reflecting part 18 and the substrate 11 (light-transmitting film 11-1) has a circular profile. The light-transmitting film 11-1 can also be selectively applied only on the light-transmitting region within the reflecting surface 18-1 (i.e., the circle). Specifically, the light-transmitting film 11-1 can be selectively applied only on the light-transmitting region through which light received by the light-receiving part 16 is transmitted.

In the example shown in FIG. 12, the light-emitting part 14 is placed on the second surface (e.g., the reverse surface) of the substrate 11. As with the first surface, the light-transmitting film 11-1 can be formed on a region on the second surface of the substrate 11 on which the light-emitting part 14 is not placed. The light-transmitting film 11-1 is preferably formed on at least the light-transmitting region (i.e., the light-transmitting region through which light emitted by the light-emitting part 14 is transmitted). In the example shown in FIG. 12, at an end part 11-2 of the substrate 11, a wiring 63 is formed on the first surface, formed so as to penetrate the substrate 11, and formed on the second surface. Similarly, at the end part 11-2 of the substrate 11, a wiring 64 is formed on the first surface, formed so as to penetrate the substrate 11, and formed on the second surface. The wiring 63 is connected to a cathode of the light-emitting part 14, on the side of the second surface, via, e.g., a bonding wire 63-1, and the wiring 64 is connected to an anode of the light-emitting part 14, on the side of the second surface, via, e.g., a bonding wire 64-1. Causing the end part 11-2 of the substrate 11 sandwiched between the reflecting part 18 and the contact part 19 to protrude outwards makes it possible to draw the wirings for the light-emitting part 14 and the light-receiving part 16 to the exterior.

FIG. 13 3 shows an example of accommodating the substrate 11. The substrate 11 can be formed from a flexible substrate. Therefore, the end part 11-2 of the substrate 11 can be bent. The substrate 11 can be connected to a motherboard 82 (e.g., a principal substrate forming the biological information measuring device described further below) of a computer in a state in which the end part 11-2 of the substrate 11 is bent, as shown in FIG. 13. Specifically, bending the substrate 11 makes it possible to provide a small biological information detector. In FIG. 13, the light-transmitting film 11-1 is not shown. The light-emitting part 14 and the light-receiving part 16 are also not shown. The wirings for the light-emitting part 14 and the wirings for the light-receiving part 16 can be formed on the substrate 11 as shown, e.g., in FIG. 12. The wirings can connect a control circuit on the motherboard 82 to the light-emitting part 14 and the light-receiving part 16 via a connector 84.

The substrate 11 is sandwiched between the reflecting part 18 and the contact part 19, and the reflecting part 18 is thereby secured to the substrate 11. One of either the light-emitting part 14 or the light-receiving part 16 can be arranged in a space formed by the reflecting surface of the reflecting part 18 and the substrate 11. While the substrate 11 to which the reflecting part 18 is secured cannot be locally bent, the end part 11-2 of the substrate 11 to which the reflecting part 18 is not secured can be bent. Since the substrate 11 is sandwiched between the reflecting part 18 and the contact part 19, the light-emitting part 14 and the light-receiving part 16 can be installed on the substrate 11 and supported even if the substrate 11 is a flexible substrate that is devoid of inherent stiffness.

### 1.11 Another example of a configuration of biological information detector

FIG. 14 shows another example of configuration of the biological information detector according to the present embodiment. As shown in FIG. 14, the biological information detector can include a reflecting part 92 for reflecting light. In the descriptions given below, the reflecting part 92 is referred to a first reflecting part 92, and the reflecting part 18 such as that shown in FIG. 1 and other drawings is referred to as the second reflecting part. Structures that are identical to those in the examples described above are affixed with the same numerals, and a description of the structures is not provided. In the example shown in FIG. 14, the first reflecting part 92 and the light-receiving part 16 are arranged after the light-transmitting film 11-1 is formed on the substrate 11.

In the example shown in FIG. 14, the biological information detector includes the light-emitting part 14, the first reflecting part 92, the light-receiving part 16, and the second reflecting part 18. The light-emitting part 14 emits a first light R1 towards the detection site O of the test subject (e.g., the user), and a second light R2 towards a direction other than that of the detection site O (i.e., towards the first reflecting part 92). The first reflecting part 92 reflects the second light R2 towards the detection site O. The light-receiving part 16 receives lights R1' and R2' having biological information (i.e., reflected light), which are, respectively, first light R1 and second light R2 reflected at the detection site O. The second reflecting part 18 reflects the lights R1' and R2' having biological information from the detection site O (i.e., reflected light) towards the light-receiving part 16. Due to the presence of the first reflecting part 92, the second light R2, which does not directly reach the detection site O of the test subject (i.e., the user), also reaches the detection site O. Specifically, the amount of light reaching the detection site O via the first reflecting part 92 increases, and the efficiency of the light-emitting part 14 increases. Therefore, the detection accuracy (i.e., the signal-to-noise ratio) of the biological information detector improves.

In the example shown in FIG. 14, the second light R2 proceeds into the interior of the test subject, and the reflected light R2' that has been reflected at the detection site O travels towards the second reflecting part 18. The biological information (i.e., the pulse rate) is also reflected in the reflected light R2' that has been reflected at the detection site O. In the example shown in FIG. 14, the first light R1 is partially reflected at a surface (skin surface) SA of the test subject. In an instance in which the detection site O is in the interior of the test subject, the biological information (i.e., the pulse rate) is not reflected in the reflected light R1" (directly reflected light) that has been reflected at the surface SA of the test subject.

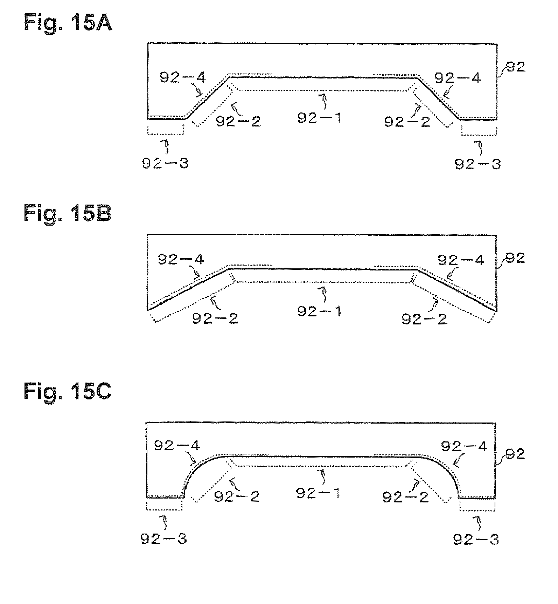

In the example shown in FIG. 14, the light-emitting part 14 can have a first light-emitting surface 14A for emitting the first light R1, disposed opposite the detection site O. The light-emitting part 14 can also have a second light-emitting surface 14B for emitting the second light R2, the second light-emitting surface 14B being a side surface of the first light-emitting surface 14A. In such an instance, the first reflecting part 92 can have a wall part surrounding the second light-emitting surface 14B, and this wall part can have a first reflecting surface (corresponding to numeral 92-2 shown in FIGS. 15A through 15C) for reflecting the second light R2 towards the detection site O. The second light R2 is not necessarily limited to that emitted from the second light-emitting surface 14B. In essence, the first reflecting surface (i.e., numeral 92-2 shown in FIGS. 15A through 15C) reflects, towards the detection site O, light (i.e., the second light R2) other than the light travelling directly towards the detection site O from the light-emitting part 14.

The wall part of the first reflecting part 92 can also have a second reflecting surface (corresponding to numeral 92-3 shown in FIGS. 15A and 15C) for reflecting light that has been reflected at the surface of the test subject and that does not have biological information (i.e., invalid light; noise), and thereby inhibiting light that does not have biological information from being incident on the light-receiving part 16.

Also, the contact part 19 is capable of ensuring there is a gap (e.g., Δh2) between the first reflecting part 92 and the detection site O. A gap (e.g., Δh2') is also present between the first reflecting part 92 and the contact part 19.

If, in cross-section view, W1 represents the maximum value of the length of the first reflecting part 92 in a direction parallel to the first surface of the substrate 11, and W2 represents the maximum value of the length of the light-receiving part 16 in that direction, a relationship W1 ≤ W2 can be satisfied. The substrate 11 transmits the reflected light R1' produced by the first light R1 emitted at the detection site O, and other light. Setting the maximum value W1 of the length of the first reflecting part 92 so as to be equal to or less than the maximum value W2 of the length of the light-receiving part 16 makes it possible to increase the amount of light reaching the second reflecting part 18. Specifically, the maximum value W1 of the length of the first reflecting part 92 can be set so that the first reflecting part 92 neither blocks nor reflects the reflected light R1' reflected at the detection site O.

FIGS. 15A, 15B, and 15C show examples of a configuration of the first reflecting part 92. As shown in FIG. 15A, the first reflecting part 92 can have a support part 92-1 for supporting the light-emitting part 14, and an inner wall surface 92-2 and a top surface 92-3 of a wall part surrounding the second light-emitting surface 14B of the light-emitting part 14. The light-emitting part 14 is not shown in FIGS. 15A through 15C. In the example shown in FIG. 15A, the first reflecting part 92 can reflect the second light R2 on the inner wall surface 92-2 towards the detection site O (see FIG. 14), the first reflecting part 92 having a first reflecting surface on the inner wall surface 92-2. The thickness of the support part 92-1 is, e.g., 50 µm to 1000 µm, and the thickness of the wall part (i.e., 92-3) is, e.g., 100 µm to 1000 µm.

In the example shown in FIG. 15A, the inner wall surface 92-2 has an inclined surface (92-2) which, with increasing distance in a width direction (i.e., a first direction) from a center of the first reflecting part 92, inclines towards the detection site O in a height direction (i.e., a direction that is perpendicular to the first direction), in cross-section view. The inclined surface (92-2) in FIG. 15A is formed by, in cross-section view, an inclined plane, but can also be a curved surface shown in e.g. FIG. 15C, or a similar inclined surface. The inner wall surface 92-2 can also be formed as a plurality of inclined planes whose angle of inclination vary from one another, or by a curved surface having a plurality of curvatures. In an instance in which the inner wall surface 92-2 of the first reflecting part 92 has an inclined surface, the inner wall surface 92-2 of the first reflecting part 92 is capable of reflecting the second light R2 towards the detection site O. Specifically, the inclined surface on the inner wall surface 92-2 of the first reflecting part 92 can be said to be the first reflecting surface for improving the directivity of the light-emitting part 14. In such an instance, the amount of light reaching the detection site O increases further. Also, the top surface 92-3 shown in FIGS. 15A and 15C can be omitted, e.g., as shown in FIG. 15B. In an instance in which the first reflecting part 92 has the top surface 92-3, reflected light R1" reflected at the surface SA of the test subject (i.e., directly reflected light) can be reflected towards the detection site O or the vicinity thereof, and this reflected light R1" is inhibited from reaching the light-receiving part 16 (see FIG. 14). Specifically, the top surface 92-3 shown in FIGS. 15A and 15C can be said to be the second reflecting surface for reflecting the directly reflected light (or in a broader sense, noise) that can otherwise reach the second reflecting part 18 and the light-receiving part 16, and reducing noise. In FIGS. 15A through 15C, a range indicated by label 92-4 functions as a mirror surface part.

In the example shown in FIG. 14, the first reflecting part 92 can protrude towards the detection site O by, e.g., a predetermined height Δh1 (e.g., Δh1 = 50 µm to 950 µm), using the surface of the light-emitting part 14 that defines the shortest distance with respect to the surface SA of the test subject (e.g., the first light-emitting surface 14A) as reference. Specifically, the gap between the first reflecting part 92 and the surface SA of the test subject (e.g., Δh2 = Δh0 ― Δh1 = 200 µm to 1200 µm) can be made smaller than the gap that represents the shortest distance between the light-emitting part 14 and the surface SA of the test subject (e.g., Δh0 = Δh1 + Δh2). Therefore, in the first reflecting part 92, the presence of, e.g., the amount of protrusion Δh1 from the light-emitting part 14 makes it possible to increase the area of the first reflecting part (i.e., 92-2) and increase the amount of light reaching the detection site O. Also, with regards to the light reflected at the detection site O, the presence of the gap Δh2 between the first reflecting part 92 and the surface SA of the test subject makes it possible to provide a light path for the light to reach the second reflecting part 18 from the detection site O. Also, in an instance in which the first reflecting part 92 has the second reflecting part (i.e., 92-3), adjusting the Δh1 and the Δh2 makes it possible to adjust the respective amounts by which light having the biological information (i.e., valid light) and light that does not have the biological information (i.e., invalid light; noise) are incident on the light-receiving part 16, and thereby further improve the signal-to-noise ratio.

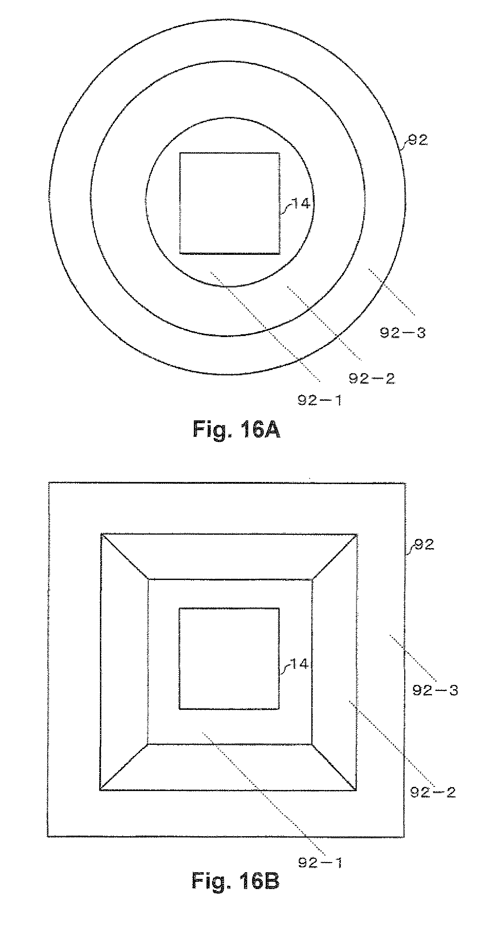

FIGS. 16A and 16B show examples of the outer appearance of the first reflecting part 92 and the light-emitting part 14 of FIG. 14 with respect to the plan view. In the example shown in FIG. 16A, with respect to the plan view (when viewed from, e.g., towards the detection site O shown in FIG. 14), the outer periphery of the first reflecting part 92 is circular, where the diameter of the circle is, e.g., 200 µm to 11,000 µm. In the example shown in FIG. 16A, the wall part (i.e., 92-2) of the first reflecting part 92 surrounds the light-emitting part 14 (see FIGS. 14 and 15A). The outer periphery of the first reflecting part 92 can also be a quadrilateral (or specifically, a square) with respect to the plan view as shown e.g. in FIG. 16B. Also, in the examples shown in FIGS. 16A and 16B, with respect to the plan view (when viewed from e.g. towards the detection site O shown in FIG. 14), the outer periphery of the light-emitting part 14 is a quadrilateral (or specifically, a square), where the length of one side of the square is e.g. 100 µm to 10,000 µm. The outer periphery of the light-emitting part 14 can also be circular

The first reflecting part 92 is made of a metal whose surface is polished to a mirror finish, and thereby has a reflective structure (or specifically, a mirror reflection structure). The first reflecting part 92 can also be formed from, e.g., a resin whose surface is subjected to mirror surface finishing. Specifically, for example, a base metal forming a base of the first reflecting part 92 is readied, and a surface of the base metal is then, e.g., subjected to plating. Alternatively, a mold of the first reflecting part 92 (not shown) is filled with a thermoplastic resin, molding is performed, and, e.g., a metal film is then deposited by vapor deposition on a surface of the mold.

In the examples shown in FIGS. 16A and 16B, with respect to the plan view (when viewed from, e.g., towards the detection site O shown in FIG. 14), a region of the first reflecting part 92 other than that directly supporting the light-emitting part 14 (i.e., the inner wall surface 92-2 and the top surface 92-3 of the wall part, and a part of the support part 92-1) is exposed. The exposed region is shown as a mirror surface part 92-4 in FIG. 15A. Although in the example shown in FIG. 15A, a dotted line representing the mirror surface part 92-4 is shown within the first reflecting part 92, the mirror surface part 92-4 is actually formed on a surface of the first reflecting part 92.

In the examples shown in FIGS. 15A, 15B, and 15C, the mirror surface part 92-4 preferably has a high reflectivity. The reflectivity of the mirror surface part 92-4 is, e.g., 80% to 90% or higher. It is possible for the mirror surface part 92-4 to be formed only on the inclined surface of the inner wall surface 92-2. In an instance in which the mirror surface part 92-4 is formed not only on the inclined surface of the inner wall surface 92-2 but also on the support part 92-1, the directivity of the light-emitting part 14 increases further. In an instance in which the mirror surface part 92-4 is formed on the top surface 92-3, the first reflecting part 92 can reflect, towards the detection site O or the vicinity thereof, the reflected light R1" reflected at the surface SA of the test subject (i.e., directly reflected light; invalid light) as shown, e.g., in FIG. 11. This reflected light R1" is inhibited from reaching the second reflecting part 18 and the light-receiving part 16. The directivity of the light-emitting part 14 increases and the amount of directly reflected light (or in a broader sense, noise) decreases, and the detection accuracy of the biological information detector therefore improves.

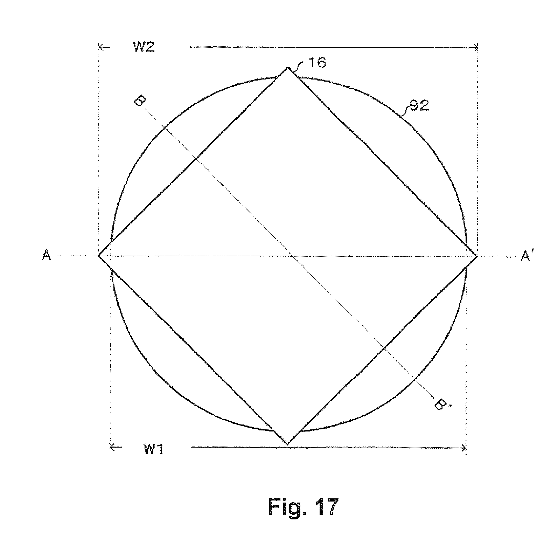

FIG. 17 shows an example of the outer appearance of the light-receiving part 16 shown in FIG. 14. In the example shown in FIG. 17, with respect to the plan view (when viewed from, e.g., the side towards the second reflecting part 18 in FIG. 14), the outer periphery of the light-receiving part 16 is a quadrilateral (or specifically, a square), where the length of one side of the square is, e.g., 100 µm to 10,000 µm. Also, with respect to the plan view (when viewed from, e.g., the side towards the second reflecting part 18 in FIG. 11), the outer periphery of the first reflecting part 92 is circular. The outer periphery of the first reflecting part 92 can be a quadrilateral (or specifically, a square) as in the example shown in FIG. 16B. The outer periphery of the light-receiving part 16 can also be circular.

In the example shown in FIG. 17, if W1 represents the maximum value of the length of the first reflecting part 92 and W2 represents the maximum value of the length of the light-receiving part 16, a relationship W1 ≤ W2 can be satisfied, as shown by the segment A-A'. A cross-section view in which the segment A-A' in FIG. 17 is used corresponds to FIG. 14. With regards to a cross-section view in which the segment B-B' in FIG. 17 is used corresponds to FIG. 14, the maximum value W1 of the length of the first reflecting part 92 is greater than the minimum value of the length of the light-receiving part 16. Although the maximum value W1 of the length of the first reflecting part 92 can be set so as to be equal to or less than the minimum value of the length of the light-receiving part 16, the efficiency of the first reflecting part 92 (or in a broader sense, the efficiency of the 14), will decrease. In the example shown in FIG. 17, the maximum value W1 of the first reflecting part 92 is set so as to be equal to or less than the maximum value W2 of the length of the light-receiving part 16, and the maximum value W1 of the length of the first reflecting part 92 is set so as to be greater than the minimum value of the length of the light-receiving part 16, so that the reflected light R1' is neither blocked nor reflected, while the efficiency of the light-emitting part 14 is maintained.

### 2. Biological information measuring device

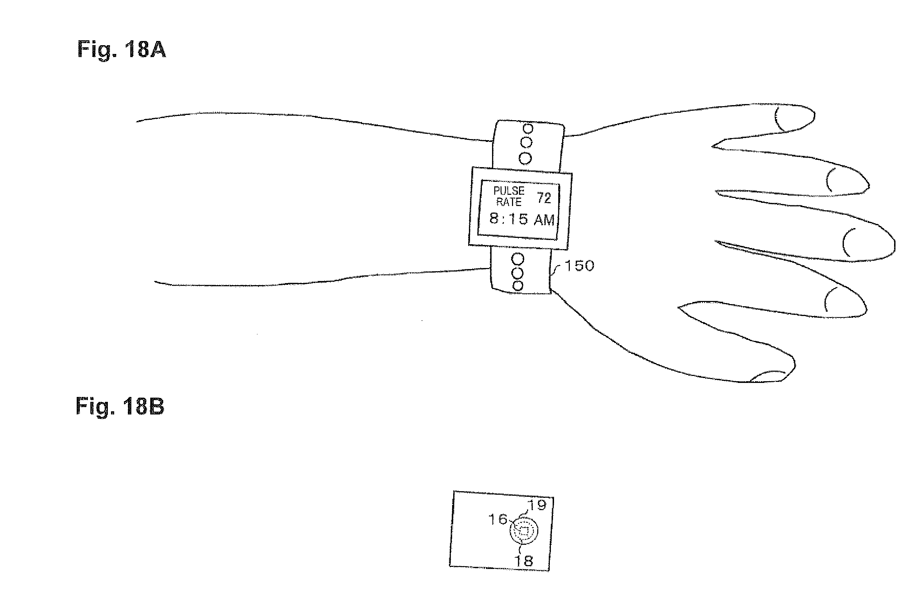

FIGS. 18A and 18B are examples of the outer appearance of a biological information measuring device including the biological information detector such as that shown in FIG. 1, and other drawings. As shown in FIG. 18A, the biological information detector shown, e.g., in FIG. 1 can further include a wristband 150 capable of attaching the biological information detector to an arm (or specifically, a wrist) of the test subject (i.e., the user). In the example shown in FIG. 18A, the biological information is the pulse rate indicated by, e.g., "72." The biological information detector is installed in a wristwatch showing the time (e.g., "8:15 am"). Also, as shown in FIG. 18B, an opening part is provided to a back cover of the wristwatch, and the contact part 19 shown in FIG. 1, for example, is exposed in the opening part. In the example shown in FIG. 18B, the second reflecting part 18 and the light-receiving part 16 are installed in a wristwatch. In the example shown in FIG. 18B, the first reflecting part 92, the light-emitting part 14, the wristband 150, and other components are omitted.

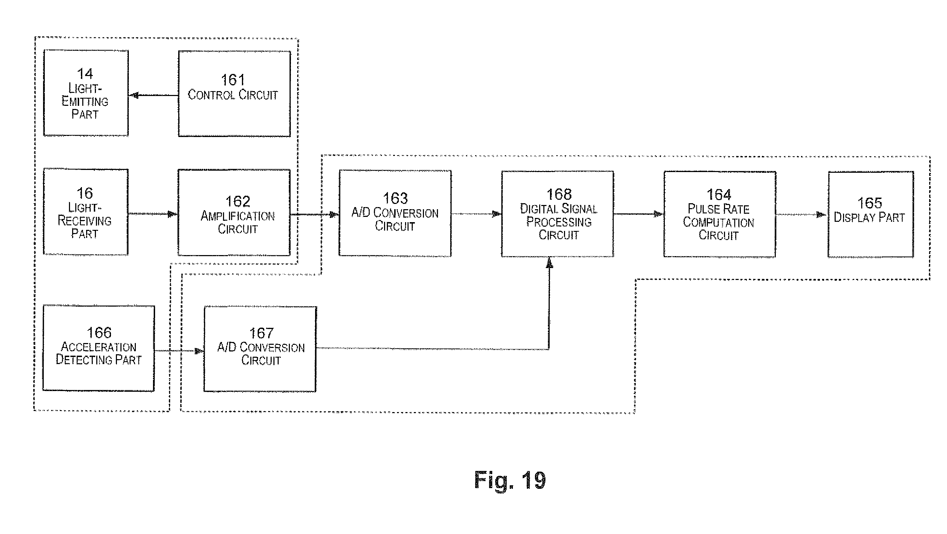

FIG. 19 is an example of a configuration of the biological information measuring device. The biological information measuring device includes the biological information detector shown in FIG. 1 and other drawings, and a biological information measuring part for measuring biological information from a light reception signal generated at the light-receiving part 16 of the biological information detector. As shown in FIG. 19, the biological information detector can have the light-emitting part 14, the light-receiving part 16, and a circuit 161 for controlling the light-emitting part 14. The biological information detector can further have a circuit 162 for amplifying the light reception signal from the light-receiving part 16. The biological information measuring part can have an A/D conversion circuit 163 for performing an A/D conversion of the light reception signal from the light-receiving part 16, and a pulse rate computation circuit 164 for calculating the pulse rate. The biological information measuring part can further have a display part 165 for displaying the pulse rate.

The biological information detector can have an acceleration detecting part 166, and the biological information measuring part can further have an A/D conversion circuit 167 for performing A/D conversion of a light reception signal from the acceleration detecting part 166 and a digital signal processing circuit 168 for processing a digital signal. The configuration of the biological information measuring device is not limited to that shown in FIG. 17. The pulse rate computation circuit 164 in FIG. 19 can be, e.g., an MPU (i.e., a micro processing unit) of an electronic device installed with the biological information detector.

The control circuit 161 in FIG. 19 drives the light-emitting part 14. The control circuit 161 is, e.g., a constant current circuit, delivers a predetermined voltage (e.g.. 6 V) to the light-emitting part 14 via a protective resistance, and maintains a current flowing to the light-emitting part 14 at a predetermined value (e.g., 2 mA). The control circuit 161 is capable of driving the light-emitting part 14 in an intermittent manner (e.g. at 128 Hz) in order to reduce consumption current. The control circuit 161 is formed on, e.g., a motherboard, and wiring between the control circuit 161 and the light-emitting part 14 is formed, e.g., on the substrate 11 shown in FIG. 1.

The amplification circuit 162 shown in FIG. 19 is capable of removing a DC component from the light reception signal (i.e., an electrical current) generated in the light-receiving part 16, extracting only an AC component, amplifying the AC component, and generating an AC signal. The amplification circuit 162 removes the DC component at or below a predetermined frequency using, e.g., a high-pass filter, and buffers the AC component using, e.g., an operational amplifier. The light reception signal contains a pulsating component and a body movement component. The amplification circuit 162 or the control circuit 161 is capable of feeding a power supply voltage for operating the light-receiving part 16 at, e.g., reverse bias to the light-receiving part 16. In an instance in which the light-emitting part 14 is intermittently driven, the power supply to the light-receiving part 16 is also intermittently fed, and the AC component is also intermittently amplified. The amplification circuit 162 is formed on, e.g., the mother board 82 shown in FIG. 13, and wiring between the amplification circuit 162 and the light-receiving part 16 is formed on, e.g., the substrate 11 shown in FIG. 1. The amplification circuit 162 can also have an amplifier for amplifying the light reception signal at a stage prior to the high-pass filter. In an instance in which the amplification circuit 162 has an amplifier, the amplifier is formed, e.g., on the end part 11-2 of the substrate 11 shown in FIG. 12.

The A/D conversion circuit 163 shown in FIG. 19 converts an AC signal generated in the amplification circuit 162 into a digital signal (i.e., a first digital signal). The acceleration detecting part 166 shown in FIG. 19 calculates, e.g., acceleration in three axes (i.e., x-axis, y-axis, and z-axis) and generates an acceleration signal. Movement of the body (i.e., the arm), and therefore movement of the biological information measuring device, are reflected in the acceleration signal. The A/D conversion circuit 167 shown in FIG. 19 converts the acceleration signal generated in the acceleration detecting part 166 into a digital signal (i.e., a second digital signal).

The digital signal processing circuit 168 shown in FIG. 19 uses the second digital signal to remove or reduce the body movement component in the first digital signal. The digital signal processing circuit 168 can be configured using, e.g., an FIR filter or another adaptive filter. The digital signal processing circuit 168 inputs the first digital signal and the second digital signal into the adaptive filter and generates a filter output signal from which noise has been removed or reduced.

The pulse rate computation circuit 164 shown in FIG. 19 uses e.g. fast Fourier transform (or in a broader sense, discrete Fourier transform) to perform a frequency analysis on the filter output signal. The pulse rate computation circuit 164 identifies a frequency that represents a pulsating component based on the result of the frequency analysis, and calculates the pulse rate.

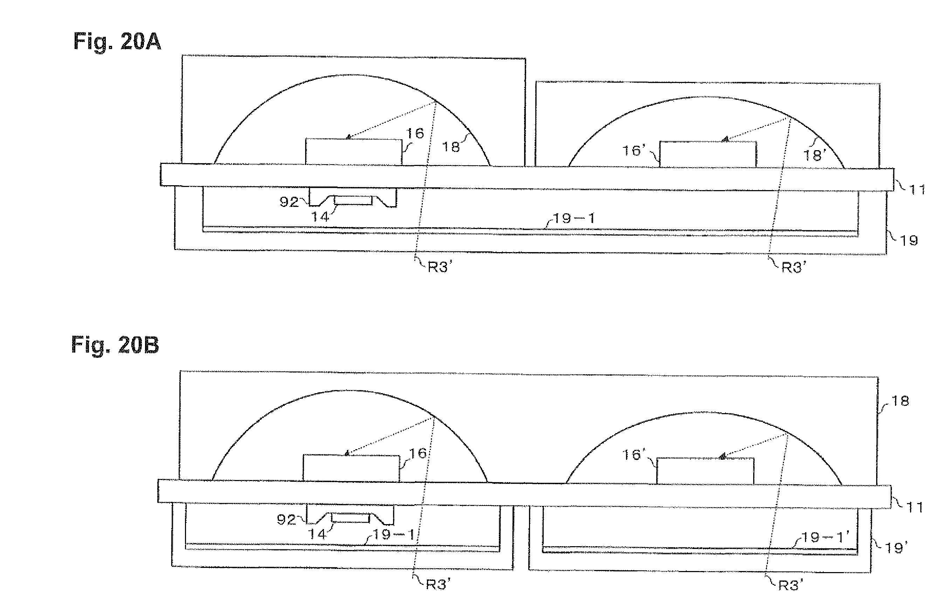

FIGS. 20A and 20B show another example of configuration of the biological information detector according to the present embodiment. The biological information detector shown in FIG. 1 and other drawings includes the filter 19-1. However, as shown in FIGS. 20A and 20B, the external light R3' (propagated light) which propagated through the test subject can be transmitted through the filter 19-1 and reach the light-receiving part 16. The external light R3 diffuses or scatters in the interior of the test subject in locations where, e.g., a tendon, a bone, or another obstacle is not present. Therefore, noise information is reflected in the propagated light R3' which propagated through the test subject. In an instance in which the effect of the propagated light R3' which propagated through the test subject cannot be disregarded, the biological information detector can include a second light-receiving part 16' such as that shown in FIGS. 20A and 20B.

In an instance in which the light-receiving part 16 is referred to as a detection sensor part (i.e., a first sensor part), the second light-receiving part 16' can be referred to as a corrective sensor part (i.e., a second sensor part). As shown in FIG. 20A, the filter 19-1 and the contact part 19 can also be provided to the corrective sensor part. In an instance in which the filter 19-1 is not provided to the corrective sensor part, a problem is presented in that the difference between the noise information received by the light-receiving part 16 and the noise information received by the second light-receiving part 16' will increase.

The biological information detector can include a reflecting part 18' such as that shown in FIG. 20A so that the propagated light R3' which propagated through the test subject reaches both the light-receiving part 16 and the second light-receiving part 16' in substantially the same condition. The reflecting part 92, the reflecting part 18, and the reflecting part 18' can be referred to as a first reflecting part, a second reflecting part, and a third reflecting part, respectively. In reality, propagated light R3' which propagated through the test subject enters the biological information detector from a variety of directions. Therefore, the presence of the first reflecting part 92 or the presence of the light-emitting part 14 can cause a part of the propagated light R3' which propagated through the test subject to be blocked or reflected by the first reflecting part 92 and the light-emitting part 14. If S1 represents the area of the reflecting surface (i.e., the domed surface) of the second reflecting part 18 and S2 represents the area of the reflecting surface of the third reflecting part 18', a relationship S2 < S 1 is preferably satisfied, as with the example shown in FIG. 20A. In an instance in which the relationship S2 < S1 is satisfied, it is possible to inhibit the propagated light R3' which propagated through the test subject from reaching the third reflecting part 18' by an amount corresponding to the reduction in the area of the reflecting surface of the third reflecting part 18' (= S1 - S2). For example, the relationship S2 < S1 can be satisfied by setting the radius of the arc or the focal distance of the parabola defining the reflecting surface of the third reflecting part 18' so as to be smaller than the radius of the arc or the focal distance of the parabola defining the reflecting surface of the second reflecting part 18.

In an instance in which the second reflecting part 18 and the third reflecting part 18' are referred to as a combined reflecting part, the second reflecting part 18 and the third reflecting part 18' can be formed from a single combined reflecting part as shown in FIG. 20B. Also, a single contact part 19 (i.e., a combined contact part) shown in FIG. 20A can be formed from the contact part 19 and a second contact part 19' as shown in FIG. 20B. Examples of the configuration of the biological information measuring device are not limited to those shown in FIGS. 20A and 20B.

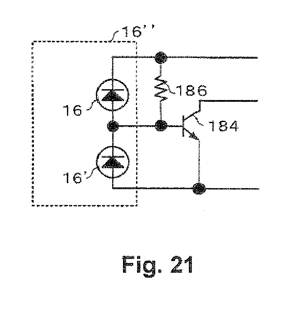

FIG. 21 shows an example of a connection between the light-receiving part 16 and the second light-receiving part 16'. As shown in FIG. 21, the anode of the light-receiving part 16 is connected to the cathode of the second light-receiving part 16', forming a combined light-receiving part 16". The anode of the light-receiving part 16 can be provided independently of the cathode of the second light-receiving part 16', and a signal generated in the light-receiving part 16 (i.e., a detection light reception signal) can be extracted independently of a signal generated in the second light-receiving part 16' (i.e., a corrective light reception signal).

In the example shown in FIG. 21, the combined light-receiving part 16" outputs a light reception signal representing the difference between the detection light reception signal and the corrective light reception signal. The detection light reception signal generated by the light-receiving part 16 contains biological information originating from light emitted by the light-emitting part 14 and noise information originating from external light, and the corrective light reception signal generated in the second light reception signal 16' contains noise information originating from external light. Therefore, the light reception signal representing the difference between the detection light reception signal and the corrective light reception signal is able to represent only the biological information originating from light emitted by the light-emitting part 14. Specifically, the combined biological information from the light-receiving part 16 (i.e., biological information from the detection site O and first noise information originating from the external light R3) can be corrected using the noise information from the second light reception signal 16' (i.e., second noise information originating from the external light R3). The first noise information is corrected or cancelled out with the second noise information, whereby the detection accuracy of the biological information detector is further improved.

Also, as shown in the example of FIG. 21, it is also possible to add a bipolar transistor (or in a broader sense, an amplifier 184) for amplifying the light reception signal from the combined light-receiving part 16", the light reception signal being inputted at the base. A resistor 186 can also be added between the anode of the light-receiving part 16 and the cathode of the second light-receiving part 16'.

Although a detailed description was made concerning the present embodiment as stated above, persons skilled in the art should be able to easily understand that various modifications are possible without substantially departing from the scope and effects of the invention. Accordingly, all of such examples of modifications are to be included in the scope of the invention. For example, terms stated at least once together with different terms having broader sense or identical sense in the specification or drawings can be replaced with those different terms in any and all locations of the specification or drawings.

## Claims

1. A biological information detector, comprising
a light-emitting part that emits light in an emission wavelength band that includes a wavelength of interest, the light being emitted towards a detection site of a test subject;
a light-receiving part that receives light having biological information, the light being light emitted by the light-emitting part and reflected at the detection site, the light-receiving part having sensitivity in a reception wavelength band that includes the emission wavelength band;
a reflecting part having a reflecting surface, the light having the biological information being reflected at the reflecting surface towards the light-receiving part; and
a filter that transmits the wavelength of interest, the filter being provided between the detection site and the light-receiving part; wherein
when an angle of incidence of incident light on the filter is a predetermined angle, a maximum wavelength of the incident light passing through the filter is higher than the wavelength of interest, and the filter inhibits light that is within a wavelength band from the maximum wavelength of the incident light to at least a maximum wavelength of the reception wavelength band; and
the reflecting surface is configured so that only light having an angle of incidence that is equal to or less than the predetermined angle reaches the light-receiving part.

2. The biological information detector according to Claim 1, wherein
the filter is a dielectric multilayer filter.

3. The biological information detector according to Claim 1 or 2, further comprising a substrate formed from a material that is transparent with respect to the wavelength of interest, the light-emitting part being arranged on a first surface, and the light-receiving part being arranged on a second surface disposed opposite the first surface; and
a contact part arranged on a side towards the first surface in relation to the substrate, the contact part being formed from a material that is transparent with respect to the wavelength of interest, and the contact part having a surface that comes into contact with the test subject; wherein
the reflecting part is arranged on a side towards the second surface in relation to the substrate.

4. The biological information detector according to Claim 3, wherein
the filter is provided to at least one of the substrate, the light-receiving part, the reflecting part, and a surface of the contact part other than the contact surface.

5. The biological information detector according to any of Claims 1 to 4, wherein
the filter is configured so that the maximum wavelength of the incident light is a wavelength corresponding to a point of change at which characteristics of an increase in the intensity of external light propagating through the test subject between a peripheral side of the detection site and a center side of the detection site shift from a first incline to a second incline that is steeper than the first incline.

6. The biological information detector according to Claim 5, wherein
the wavelength of the point of change is in a range of 565 nm to 595 nm.

7. A biological information measuring device, comprising
the biological information detector according to any of Claims 1 through 6,
a biological information measuring part that measures, from a light reception signal generated in the light-receiving part, the biological information; wherein
the biological information is a pulse rate.
